# EUROPEAN PATENT SPECIFICATION

(11) **EP 1 814 993 B1**
(45) Date of publication and mention of the grant of the patent: **06.01.2010**
(21) Application number: 05806365.2
(22) Date of filing: 31.10.2005
(51) Int. Cl.: C12N 15/62

(54) **MULTIFUNCTIONAL AND MULTIVALENT ANGIOGENESIS INHIBITORS**
MULTIFUNKTIONELLE UND MULTIVALENTE ANGIOGENESE-INHIBITOREN
INHIBITEURS DE L'ANGIOGENESE MULTIFONCTIONNELS ET POLYVALENTS

(30) Priority: 02.11.2004 ES 200402635 P
(43) Date of publication of application: 08.08.2007
(73) Proprietor: Universidad Autónoma de Madrid, 28049 Madrid (ES)
(72) Inventor: ÁLVAREZ VALLINA, Luis, E-28035 Madrid (ES); SÁNCHEZ-ARÉVALO LOBO, Víctor Javier, E-28035 Madrid (ES); CUESTA MARTÍNEZ, Ángel, E-28035 Madrid (ES); SANZ ALCOBER, Laura, E-28035 Madrid (ES); VARGAS NUÑEZ, Juan, E-28035 Madrid (ES); COMPTE GRAU, Marta, E-28035 Madrid (ES)
(74) Representative: ABG Patentes, S.L.
(86) International application number: PCT/EP2005/011714
(87) International publication number: WO 2006/048252

(56) References cited:
- WO-A-00/11033
- US-A1- 2003 054 409
- US-A1- 2003 139 365
- US-A1- 2004 081 647
- US-A1- 2004 081 648
- US-A1- 2004 120 955
- US-B1- 6 495 346
- SANZ LAURA ET AL: "Antibody engineering: facing new challenges in cancer therapy" ACTA PHARMACOLOGICA SINICA, vol. 26, no. 6, June 2005 (2005-06), pages 641-648, XP002364460 ISSN: 1671-4083
- SANZ LAURA ET AL: "Generation and characterization of recombinant human antibodies specific for native laminin epitopes: Potential application in cancer therapy" December 2001 (2001-12), CANCER IMMUNOLOGY IMMUNOTHERAPY, VOL. 50, NR. 10, PAGE(S) 557-565 , XP002365676 ISSN: 0340-7004

## Description

### FIELD OF THE INVENTION

The invention is related to fusion proteins useful as multifunctional and multivalent angiogenesis inhibitors comprising a polypeptide which recognizes and blocks a functionally active region of a molecule involved in the angiogenesis process, and a polypeptide comprising an oligomerization domain and a functionally active, inhibitor or modulator region of the angiogenesis process, separated by a proteinase-sensitive region. The invention also relates to gene and vector constructs useful for producing said fusion proteins.

### BACKGROUND OF THE INVENTION

Angiogenesis is the biological process leading to new blood vessel formation from the pre-existing vessels in an organ or tissue. Angiogenesis begins with the decomposition of the basal membrane due to the action of the proteinases secreted by endothelial cells, continues with the migration and proliferation of said endothelial cells, to conclude with the formation of the lumen, the basal membrane and the envelopment with peripheral cells.

Angiogenesis does not exist under normal physiological conditions in a healthy adult, with the exception of the phenomena associated with the female menstrual cycle and wound healing. However, an imbalance in the angiogenesis process contributes to the development of pathological disorders, such as rheumatic arthritis, psoriasis, bartonellosis, transplanted organ rejection, hemorrhaging and ocular neovascularization (one of the most frequent cases of blindness), diabetic retinopathy, retinopathy of prematurity, macular degeneration, neovascular glaucoma, retinal vein occlusion, retinal artery occlusion, pterygium, rubeosis, corneal neovasculature, solid tumors, hemangioma and tumor proliferation and metastasis, among others.

Angiogenesis further plays an important role in the progressive growth and metastatic spreading of tumors. A tumor must continually stimulate the development of new capillaries in order to be able to grow. The new vessels generated in the tumor provide malignant cells with a pathway through which they may enter circulation and form metastasis in distant sites. If this angiogenic activity could be suppressed or eliminated, then even though the tumor is present, it could not develop.

For this reason, different research groups are working to find compounds exercising an inhibitory action on angiogenesis (angiogenesis inhibitors or antiangiogenic agents) useful as therapeutic agents for treating or preventing pathologies occurring with the development of angiogenesis.

Regarding tumors, it is accepted that tumors cannot grow or metastasize to another organ without the genesis of new blood vessels, thus constituting the angiogenic switch an early event in tumor progression. Several antiangiogenic strategies interfering at different levels of the angiogenic pathway have been described: blocking growth factor activity; inhibiting extracellular matrix (ECM) proteinases; leading directly at endothelial cells (EC); excessively regulating endogenous inhibitors, etc. Endogenous angiogenesis inhibitors have received special attention in cancer therapy since it seems that they are non-toxic and non-immunogenic agents. At least 10 endogenous angiogenesis inhibitors have been identified [O'Reilly, M.S. et al., Cell, 88: 277-285, 1997], of which the most well known are angiostatin and endostatin.

Endostatins (ES) are endothelial cell migration and angiogenesis inhibitors, and it has been demonstrated that they reduce tumor growth in animal models. The mechanisms involved in said effects are not clear, though it has been proposed that they are involved in cell surface receptor binding (integrins and heparan sulfates, VEGFR-2), metalloproteinases and ECM components. ES derive from the NCI domain of collagens XV and XVIII, from where they are proteolytically released in trimeric form and they are further converted into monomeric endostatins of about 20 kDa. At the NC1 N-terminal end, there is a trimerization domain of about 60 residues connected with the ES module of 180 residues approximately by means of a flexible hinge region containing different proteinase-sensitive sites releasing endostatin after the proteolytic cleavage.

Boehm et al. [Nature, 390:404 (1997)] describe the use of, as an experimental model, mice in which several tumors (Lewis's pulmonary carcinoma, fibrosarcoma and melanoma) have been transplanted. In the mice not treated with ES, said tumors grew rapidly, causing the death of the animal. In contrast, the mice treated with ES after tumor development, a reduction of the tumor volume until reaching an almost microscopic size was observed. The cyclical treatment with ES of tumor-carrying mice caused a complete regression of the tumors in animal models. However, clinical trials being carried out with ES have not reported a significant tumor growth inhibition and tumor regression has rarely been observed. Unfortunately, this is not an isolated example and other clinical trials including different anti-angiogenic molecules are also disappointing, despite how significant the antitumor effects in animal models were. In this context it seems logical to combine several angiogenesis inhibitors for treating human cancers. Alternatively, the enhanced biological activity (ES + angiostatin) and the action aimed at the tumor [ES + RGD (arginine-glycine-aspartic acid)] may potentially improve tumor growth inhibition.

On the other hand, the therapeutic potential of a single chain Fv (scFv) fragment of an anti-laminin antibody (L36) with antiangiogenic activity has been demonstrated both *in vivo* and *in vitro.* The.results showed that the alteration of the morphogenetic potential of the matrixes associated to the cells is an effective way of preventing *in vivo* the formation of blood vessels associated to the tumor.

Despite the efforts made until now, it is still necessary to develop angiogenesis inhibitor compounds useful as therapeutic agents for treating or preventing pathologies occurring with angiogenesis development.

### SUMMARY OF THE INVENTION

The invention faces the problem of providing compounds with antiangiogenic activity potentially useful as therapeutic agents for preventing and/or treating pathologies occurring with angiogenesis, for example psoriasis, rheumatic arthritis, retinopathies, cancer, etc.

The solution provided by this invention is based on fusion proteins, potentially useful as multifunctional and multivalent angiogenesis inhibitors, comprising (a) a polypeptide comprising a region which recognizes and blocks a functionally active region of a molecule involved in the angiogenesis process, and (b) a polypeptide comprising an oligomerization domain and a functionally active, inhibitor or modulator region on the angiogenesis process, separated by a proteinase-sensitive region.

The invention is illustrated by means of the construction of several proteinaceous chimeras. One of the fusion proteins (see the Example) comprises the single chain Fv (scFv) fragment of the anti-laminin monoclonal antibody L36 and the NC1 domain of collagen XVIII comprising a trimerization domain and an endostatin (ES) domain bound by a hinge peptide containing proteinase-sensitive sites releasing monomeric ES after proteolytic cleavage. Proteinase levels are increased in the tumor micro-surroundings, therefore said fusion protein releases *in situ* both ES monomers and scFv trimers. Said fusion protein was secreted by genetically modified human cells in a functionally active form. The intact form has a molecular mass of approximately 210 kDa, which indicates that, in physiological conditions, individual subunits are covalently associated in a non-covalent manner to produce a trimeric structure. Said trimeric fusion protein considerably inhibited the capacity of endothelial cells to migrate in response to growth factors and to become developed in capillary-type tubules when they grew on Matrigel substrates. Said fusion protein was likewise treated with several different proteinases. The data show that cathepsin L, pancreatic elastase and several matrix metalloproteinases (MMP) generate both ES-type monomers and trimeric antibody fragment (scFv). The fusion proteins were further processed correctly when they were produced by genetically modified MMP-producing tumor cells but not when they were produced by genetically modified cells that did not produce MMPs. These results open the path for a new gene therapy strategy against cancer using this type of fusion proteins, which form part of a new generation of angiogenesis inhibitors useful as therapeutic agents for treating diseases associated to imbalances in said angiogenesis.

Therefore, in one aspect the invention relates to a gene construct comprising, operatively bound, at least one nucleic acid sequence (A), comprising the nucleotide sequence encoding a polypeptide which recognizes and blocks a functionally active region of a molecule involved in the angiogenesis process; and a nucleic acid sequence (B) comprising the nucleotide sequence encoding a polypeptide comprising (i) an oligomerization domain, (ii) a functionally active region in the angiogenesis process, and (iii) a proteinase-sensitive region between said oligomerization domain and said functionally active region in the angiogenesis process, where the 3' end of said first nucleic acid sequence (A) is bound to the 5' end of said second nucleic acid sequence (B).

In another aspect, the invention relates to an expression cassette comprising said gene construct, operatively bound to an expression control sequence.

In another aspect, the invention relates to a recombinant vector comprising said gene construct or said expression cassette.

In another aspect, the invention relates to a host cell comprising said gene construct, or said expression cassette, or said recombinant vector.

In another aspect, the invention relates to a fusion protein obtainable by expression of the nucleic acid sequence contained in said gene construct. Said fusion protein typically comprises a polypeptide (A') comprising a region which recognizes and blocks a functionally active region of a molecule involved in the angiogenesis process; and a polypeptide (B') comprising (i) an oligomerization domain, (ii) a functionally active region in the angiogenesis process, and (iii) a proteinase-sensitive region between said oligomerization domain and said functionally active region in the angiogenesis process.

In another aspect, the invention relates to a pharmaceutical composition comprising either said fusion protein together with at least one pharmaceutically acceptable excipient, or a vector comprising a gene construct of the invention, or an expression cassette of the invention, and optionally, at least one pharmaceutically acceptable excipient.

In another aspect, the invention relates to the use of said fusion protein, or of said gene construct or of said expression cassette or of said recombinant vector, in the manufacture of a pharmaceutical composition to prevent, treat, impede or minimize the development of angiogenesis.

In another aspect, the invention relates to the use of said fusion protein or of said gene construct or of said expression cassette or of said recombinant vector in the manufacture of a pharmaceutical composition for treating and/or preventing pathologies occurring with angio genesis.

### BRIEF DESCRIPTION OF THE FIGURES

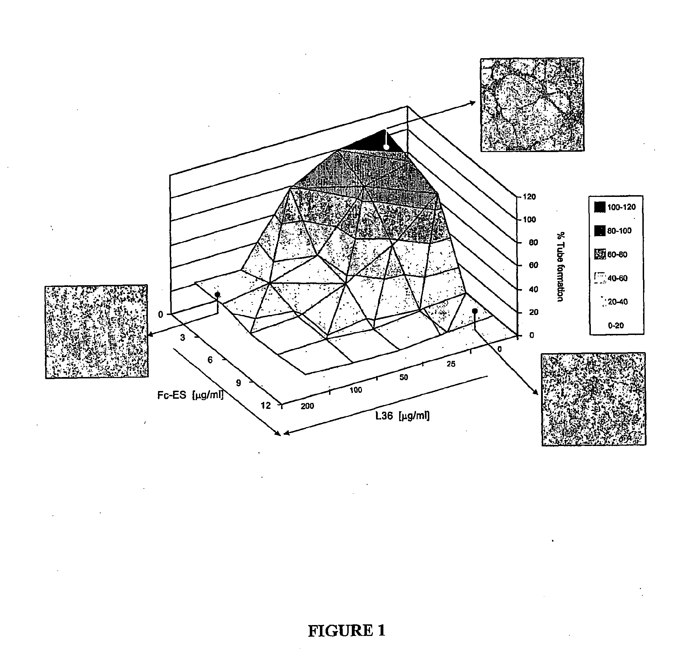

Figure 1 is a cartographic representation showing the effect on capillary morphogenesis of the individual treatment and combined treatment with different concentrations of a recombinant monoclonal antibody (L36) with scFv format [single chain Fv fragment of the anti-laminin monoclonal antibody L36] and of a dimeric Fc-ES fusion protein (fusion protein between the ES domain and the Fc fragment of an immunoglobulin).

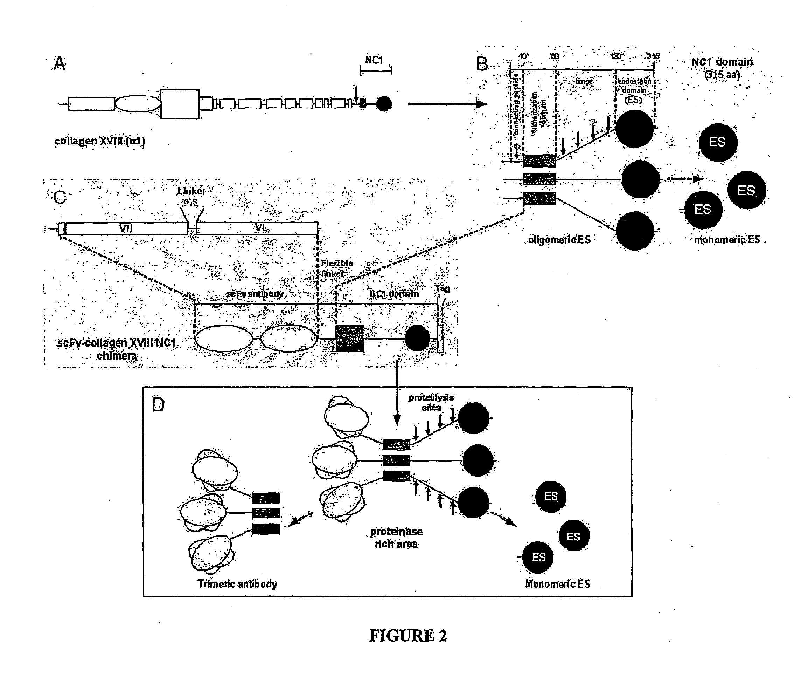

Figure 2 illustrates the production of a fusion protein provided by this invention and its proteolytic processing. Figure 2A schematically shows the gene al (collagen XVIII) and the NC1 domain encoding sequence. Figure 2B schematically shows said NC1 domain comprising a connector peptide, the trimerization domain, the hinge peptide and the ES domain. Figure 2C schematically shows a fusion protein or chimera formed by a recombinant anti-laminin monoclonal antibody (L36) with scFv format and the NC1 domain of collagen XVIII. Figure 2D shows the result of the proteolytic processing of said fusion protein, the formation of a trimeric and monomeric ES antibody is observed.

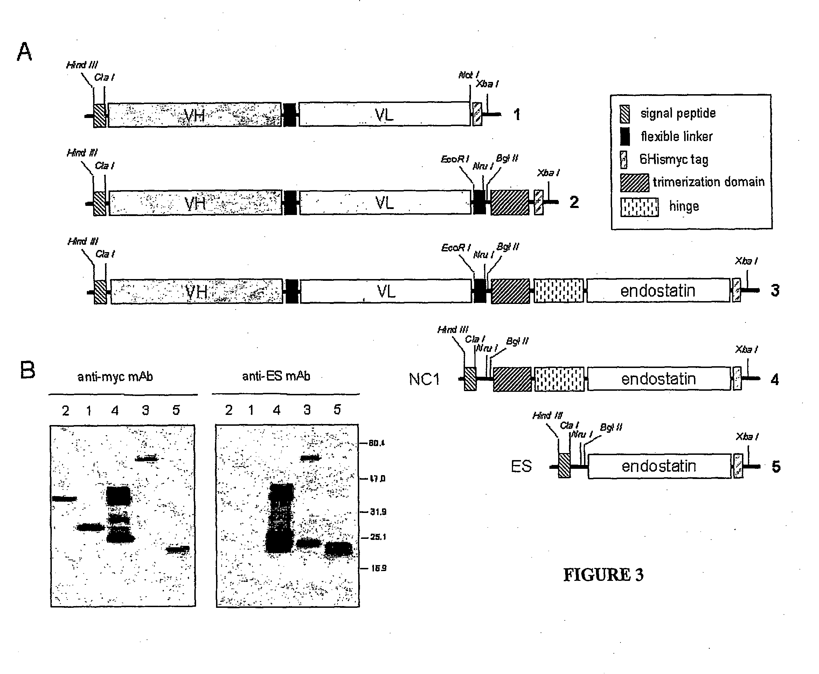

Figure 3A schematically shows the gene structure of an antibody, in scFv format and several gene constructs containing (or not) the ES domain encoding sequence. Figure 3B shows the results of a Western Blot analysis of purified fusion proteins; the immunoblotting was developed with an anti-myc monoclonal antibody (mAb) and/or with an anti-ES mAb [lane 1: L36 scFv, lane 2: L36 scFv-NC1^{ES-}, lane 3: L36 scFv-NC1^{ES+}, lane 4: NC1 and lane 5: ES].

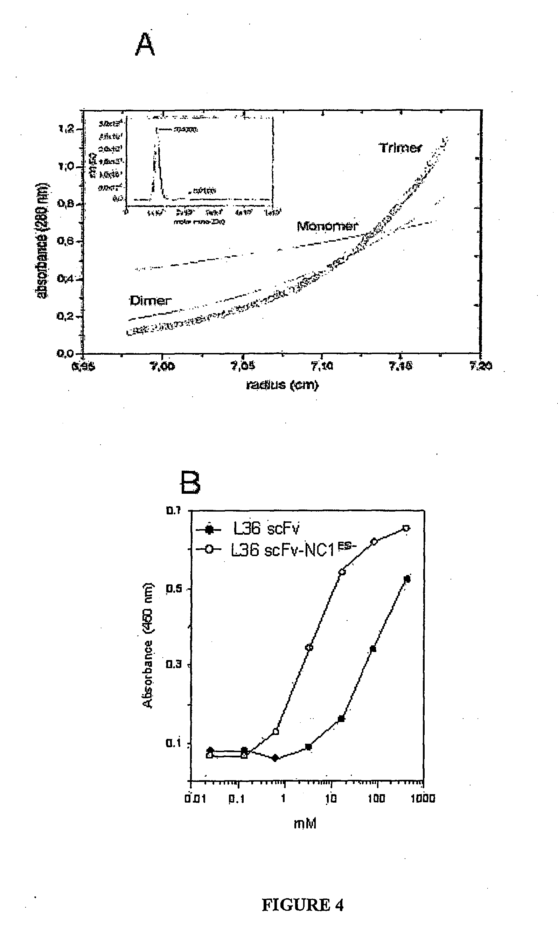

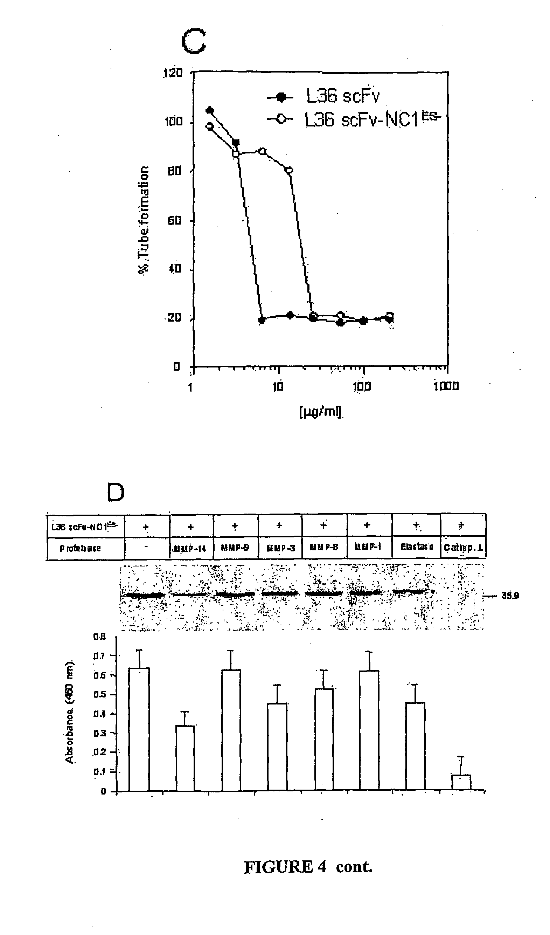

Figure 4A shows a sedimentation equilibrium gradient of the scFv-NC1^{ES-} domain (1 mg/ml in phosphate buffered saline (PBS) solution) at 11,000 rpm and 20°C. The open circles represent data, the three solid lines represent the theoretical gradients of a scFv monomer (37,148 Da), dimer (74,296 Da) and trimer (111,444 Da). Shown in the upper box of said Figure 4A, is the distribution of the sedimentation rate of the fusion protein L36 scFv-NC1^{ES-} (1 mg/ml in PBS buffer) at 42,000 rpm and 20°C. Figure 4B is a graph showing the laminin binding affinity immobilized on a plastic support [greater in the case of fusion protein L36 scFv-NC1^{ES-} (trimeric) than in the case of L36 scFv (monomeric)]. Figure 4C is a graph showing the dose-dependent modulation of the endothelial cell differentiation (in response to different scFv or L36 scFv-NC1^{ES-} concentrations) in a typical Matrigel assay. Each dot represents the average of two wells +/- standard deviation. Figure 4D shows the results of subjecting fusion protein L36 scFv-NC1^{ES-} to the action of different proteinases (MMPs, porcine pancreatic elastase and cathepsin L).

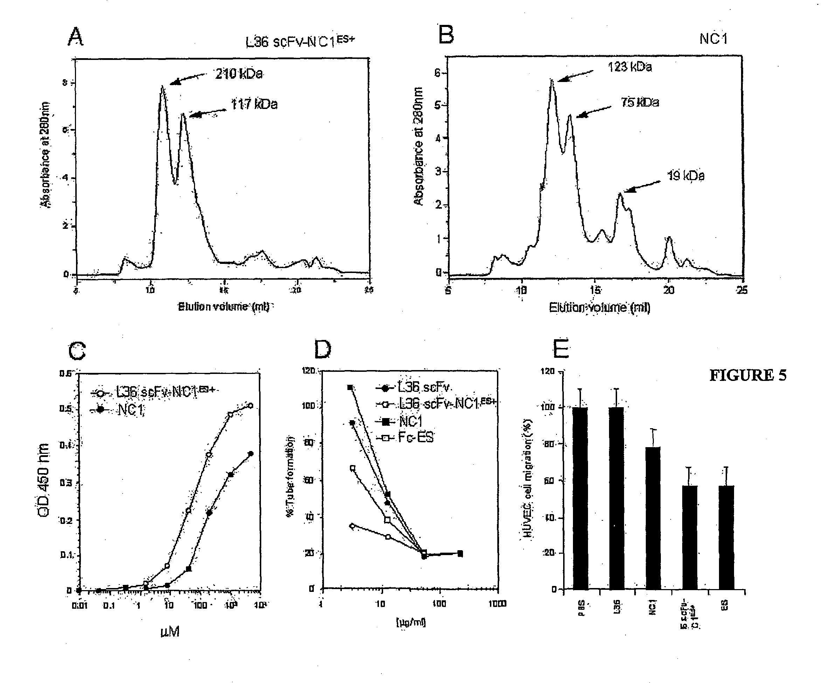

Figure 5 shows the distribution of the sedimentation rate of L36 scFv-NC1^{ES+} (Figure 5A) and NC1 (Figure 5B). Figure 5C shows the saturation curves obtained with the indicated concentrations of L36 scFv-NC1^{ES+} and NC1. Figure 5D is a graph representing the dose-dependent modulation of endothelial cell differentiation (in response to different concentrations of L36 scFv, L36 scFv-NC1E^{ES+}, NC1 or Fc-ES) in a typical Matrigel assay. Each dot represents the average of two wells +/- standard deviation. Figure 5E is a bar diagram showing modulation in a HUVEC endothelial cell assay by means of L36 scFv (L36), NC1, L36 scFv-NC1^{ES+} or Fc-ES (ES) [PBS was used as a control].

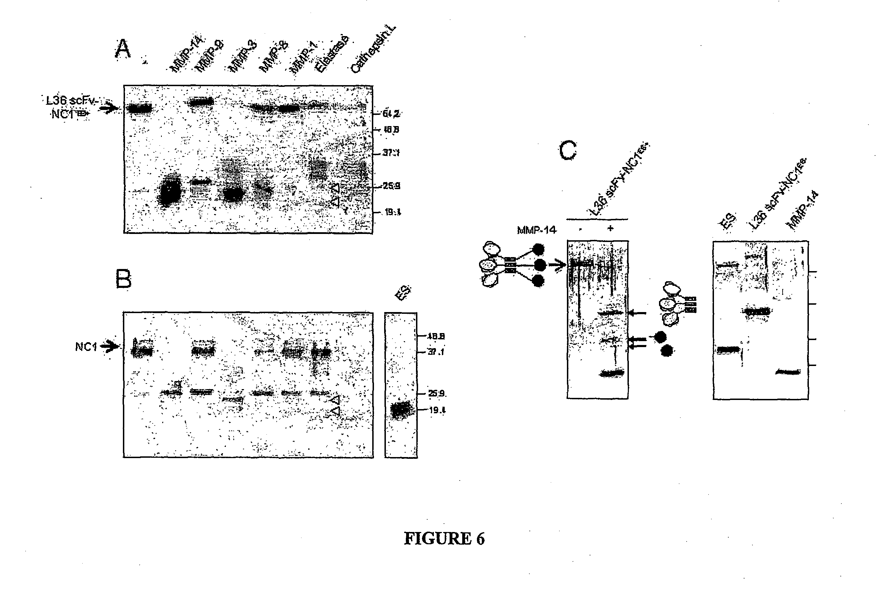

Figure 6A shows the result of treating L36 scFv-NC1^{ES+} with different proteinases (MMPs, porcine pancreatic elastase and cathepsin L), whereas Figure 6B shows the results of the same treatment for NC1. The purified proteins were incubated during the indicated times with the different proteinases as mentioned in the Example (see the Materials and Methods section) and the reaction mixtures were analyzed by means of SDS-PAGE (Figure 6C) or by means of Western Blot with and anti-endostatin mAb. The migration distances of the molecular weight and NC1 markers are indicated to the left of said figures. The empty arrow points in Figures 6A and 6B indicate the proteolytic products.

### DETAILED DESCRIPTION OF THE INVENTION

In one aspect, the invention is related to a gene construct, hereinafter gene construct of the invention, comprising, operatively bound, at least:
a) a first nucleic acid sequence (A), comprising the nucleotide sequence encoding a polypeptide which recognizes and blocks a functionally active region of a molecule involved in the angiogenesis process; and
b) a second nucleic acid sequence (B), comprising the nucleotide sequence encoding a polypeptide comprising (i) an oligomerization domain, (ii) a functionally active region in the angiogenesis process, and (iii) a proteinase-sensitive region between said oligomerization domain and said functionally active region in the angiogenesis process,
wherein the 3' end of said first nucleic acid sequence (A) is bound to the 5' end of said second nucleic acid sequence (B).

The nucleic acid sequence (A) comprises the nucleotide sequence encoding a polypeptide which recognizes and blocks a functionally active region of a molecule involved in the angiogenesis process. Illustrative examples of molecules involved in the angiogenesis process include extracellular matrix (ECM) proteins, angiogenic factors, cell membrane receptors, etc. Included among ECM proteins are collagen, proteoglycans, fibronectin, laminin, tenascin, entactin and thrombospondin. In a particular embodiment, said molecule involved in the angiogenesis process is a laminin, such as a mammal laminin, for example a rat, mouse or human laminin. Included among the angiogenic factors is the family of vascular endothelial growth factors (VEGF), etc. The receptors of said angiogenic factors, for example the VEGF receptor 2 (VEGFR-2), integrins, etc., can likewise be cited among the cell membrane receptors involved in angiogenesis.

Virtually any polypeptide comprising a region which recognizes and blocks a functionally active region of a molecule involved in the angiogenesis process may be used in this invention, such as an antibody, for example a monoclonal or polyclonal antibody, which recognizes a molecule involved in the angiogenesis process, or a recombinant fragment thereof containing the region which recognizes said molecule involved in the angiogenesis process, for example, in its recombinant single chain format (single chain Fv fragment or scFv), bifunctional (diabody), whole (Fab + Fc), etc.; nevertheless, in a particular embodiment, the nucleic acid sequence (A) encodes for a recombinant single chain antibody (scFv) derived from anti-laminin mAb L36 (Example 1) containing the variable heavy chain region (VH) of the monoclonal antibody L36 fused, through a linker, such as a peptide comprising the G₄S sequence, to the variable light chain (VL) region of mAb L36 (Figure 2C), whose sequence has been described by Sanz L et al. [Cancer Immunology and Immunotherapy, 2001 Dec; 50(10)557-65], wherein the 3' end of the VL encoding sequence is bound to the 5' end of the sequence encoding said linker and the 3' end of the nucleotide sequence encoding said linker is bound to the 5' end of the VL encoding sequence.

mAb L36 recognizes laminins of different animal species, for example, from mice, rats, humans, etc., since it interacts with a region that is very preserved among different animal species [Sanz L et al. EMBO J 2003, Vol. 22(7):1508-1517].

Due to its properties, said polypeptide which recognizes and blocks a functionally active region of a molecule involved in the angiogenesis process, such as an antibody, in any of its formats (scFv, bifunctional or complete) can recognize and block a functionally active region of a molecule involved in the angiogenesis process, and can direct an antiangiogenic polypeptide fused to said polypeptide to the molecules involved in the angiogenesis process, for example, ECM proteins, angiogenic factors, cell membrane receptors, etc., and block functionally active regions of said molecules.

The nucleic acid sequence (B) comprises the nucleotide sequence encoding a polypeptide comprising (i) an oligomerization domain, (ii) a functionally active region in the angiogenesis process, and (iii) a proteinase-sensitive region between said oligomerization domain (i) and said functionally active region in the angiogenesis process (ii).

The oligomerization domain is a domain that allows oligomer formation (e.g., dimers, trimers, tetramers, etc., of peptides or proteins). Virtually any oligomerization domain, for example a dimerization, trimerization, or tetramerization domain, etc. present in different proteins, both eukaryotic and prokaryotic in origin, capable of being expressed recombinantly and forming a proteinaceous oligomer of the protein that comprises it, can be used for putting the invention into practice. In a particular embodiment, said oligomerization domain is a trimerization domain, such as the trimerization domain of the NC1 domain of collagen XVIII or collagen XV.

The functionally active region in the angiogenesis process comprises any functionally active peptide or protein in the angiogenesis process, such as an angiogenesis process inhibitor or modulator peptide or protein. Virtually any functionally active peptide or protein in the angiogenesis process can be used in the present invention; nevertheless, in a particular embodiment, said functionally active region in the angiogenesis process comprises a mammal endostatin (ES), such as the ES present in the NC1 domain of collagen XV or collagen XVIII.

The proteinase-sensitive region is located between said oligomerization domain (i) and said functionally active region in the angiogenesis process (ii), and comprises a polypeptide sequence susceptible to the action of proteinases. Although any peptide sequence susceptible to any proteinase could be used in the invention, in practice it is advantageous for said proteinase to be a proteinase expressed only in the tumor surroundings, so that the fusion protein of the invention will be "processed", generating functionally active peptide or protein monomers in the angiogenesis process and polypeptide trimeric molecules capable of recognizing and blocking a functionally active region of a molecule involved in the angiogenesis process. Therefore, in a particular embodiment, said proteinase-sensitive region comprises the hinge region present in the NC1 domain of mammal collagen XV or XVIII, between the trimerization domain and the ES domain or region of said NC1 domain, since said region is sensitive to the action of proteinases expressed only in the tumor surroundings.

Therefore in a particular and preferred embodiment, the nucleic acid sequence (B) comprises the nucleotide sequence encoding the NC1 domain of mammal collagen XVIII, or for the NC1 domain of mammal collagen XV. As it is known, said NC1 domain of collagen XVIII (and of collagen XV) comprises a trimerization domain and an ES domain bound by a hinge peptide (Figure 2B). The sequences of said NC1 domains of collagen XV and XVIII are known; by way of illustration, the sequence of the NC1 domain of collagen XVIII has been disclosed previously by Sasaki et al. [Sasaki et al. Structure, function and tissue forms of the C-terminal globular domain of collagen XVIII containing the angiogenesis inhibitor endostatin. EMBO J. 1998 Aug 3;17(15):4249-56]. Example 1 describes obtaining the nucleotide sequence comprising the region encoding the NC1 domain of mouse collagen XVIII.

Any other domain similar to the NC1 domain of collagen XVIII comprising (i) an oligomerization domain, (ii) a functionally active region in the angiogenesis process, and (iii) a proteinase-sensitive region between said oligomerization domain and said functionally active region in the angiogenesis process, advantageously, a region sensitive to the proteinases expressed in the tumor surroundings, can be used to put this invention into practice.

Generally, the nucleic acid sequence (A) is not fused directly to the nucleic acid sequence (B), but it is advantageous to introduce a flexible binding peptide (or spacer peptide) between the polypeptides encoded by said nucleic acid sequences (A) and (B). Therefore, if desired, the gene construct of the invention may also additionally contain a third nucleic acid sequence (C) containing the nucleotide sequence encoding a flexible binding peptide located between said nucleic acid sequences (A) and (B), wherein the 5' end of said nucleic acid sequence (C) is bound to the 3' end of said nucleic acid sequence (A) and the 3' end of said nucleic acid sequence (C) is bound to the 5' end of said nucleic acid sequence (B). Advantageously, said spacer peptide (C) is a peptide with structural flexibility. Virtually any peptide with structural flexibility can be used. By way of illustration, said flexible peptide may contain repetitions of amino acid residues, particularly Gly and Ser residues, or any other suitable repetition of amino acid residues. Virtually any peptide sequence defining a flexible binding peptide can be used in this invention. Illustrative examples of flexible binding proteins include sequences such as Gly-Ser-Pro-Gly (GSPG) or sequence (Gly-Ser)₄. Nevertheless, in a particular embodiment said flexible binding protein comprises the sequence Leu-Glu-Gly-Ala-Gly-Gly-Ser-Gly-Gly-Ser-Ser-Gly-Ser-Asp-Gly-Ala-Ser-Gly-Ser. Therefore, in a particular embodiment the gene construct of the invention comprises, in addition to said nucleic acid sequences (A) and (B), a third nucleic acid sequence (C) comprising the nucleotide sequence encoding the peptide Leu-Glu-Gly-Ala-Gly-Gly-Ser-Gly-Gly-Ser-Ser-Gly-Ser-Asp-Gly-Ala-Ser-Gly-Ser.

Also, for the purpose of facilitating the isolation and purification of the fusion protein obtained by means of the present invention, the gene construct of the invention may contain, if so desired, a nucleic acid sequence encoding a peptide capable of being used for fusion protein isolation or purification purposes. Therefore, in a particular embodiment the gene construct of the invention includes, if so desired, a nucleic acid sequence (D) containing the nucleotide sequence encoding a peptide capable of being used for isolation or purification purposes, known as a tag peptide. Said nucleic acid sequence (D) can be located in any position that does not alter the functionality of any of the polypeptides expressed by said nucleic acid sequences (A) and (B). By way of illustration, said nucleic acid sequence (D) can be located downstream from the 3' end of said nucleic acid sequence (B). Virtually any peptide or peptide sequence that allows fusion protein isolation or purification can be used, for example, polyhistidine sequences, peptide sequences capable of being recognized by antibodies that may be used to purify the resultant fusion protein by immunoaffinity chromatography, such as tag peptides, etc., for example epitopes derived from the hemagglutinin of the fever virus or C-myc epitope, etc.

The gene construct of the invention can be obtained by means of the use of well known techniques in the state of the art [Sambrook et al., "Molecular cloning, a Laboratory Manual", 2nd ed., Cold Spring Harbor Laboratory Press, N.Y., 1989 Vol 1-3]. Said gene construct of the invention may incorporate, operatively bound, a regulatory sequence of the expression of the nucleic acid sequences encoding polipeptides encoded by the nucleic acid sequences (A) and (B), thus constituting an expression cassette. As used in this description, the expressional "operatively bound" means that the polypeptides encoded by the nucleic acid sequences (A) and (B), and, where appropriate (C), are expressed in the correct open reading frame under the control of the expression control or regulatory sequences.

Therefore, in another aspect, the invention provides an expression cassette comprising the gene construct of the invention operatively bound to an expression control sequence of the nucleotide sequence encoding the fusion protein provided by this invention, comprising (a) a polypeptide which recognizes and blocks a functionally active region of a molecule involved in the angiogenesis process, and (b) a polypeptide comprising (i) an oligomerization domain, (ii) a functionally active region in the angiogenesis process, and (iii) a proteinase-sensitive region between said oligomerization domain and said functionally active region in the angiogenesis process. Control sequences are sequences that control and regulate transcription and, where appropriate, the translation of said fusion protein, and include promoter sequences, sequences encoding transcriptional regulators, ribosome binding sequences (RBS) and/or transcription terminator sequences. In a particular embodiment, said expression control sequence is functional in prokaryotic cells and organisms, for example bacteria, etc., whereas in another particular embodiment, said expression control sequence is functional in eukaryotic cells and organisms, for example, insect cells, plant cells, mammal cells, etc. Illustrative examples of promoters that may be present in the expression cassette provided by this invention include the human cytomegalovirus (hCMV) promoter, etc.

Advantageously, said expression cassette further comprises a marker or gene encoding a motive or for a phenotype allowing the selection of the host cell transformed with said expression cassette. Illustrative examples of said markers that could be present in the expression cassette of the invention include antibiotic-resistant genes, toxic compound-resistant genes, and generally all those genes that allow selecting the genetically transformed plants.

The gene construct of the invention, or the expression cassette provided by this invention can be inserted in a suitable vector. Therefore, in another aspect, the invention is related to a vector, such as an expression vector, comprising said gene construct of the invention or said expression cassette. The choice of the vector will depend on the host cell where it will subsequently be introduced. By way of illustration, the vector in which said nucleic acid sequence is introduced can be a plasmid or a vector which, when introduced in a host cell, either becomes integrated or not in the genome of said cell. Said vector can be obtained by conventional methods known by persons skilled in the art [Sambrok et al., 1989, cited above]. In a particular embodiment, said recombinant vector is a vector that is useful for transforming animal cells.

Said vector can be used to transform, transfect or infect cells capable of being transformed, transfected or infected by said vector. Said cells can be prokaryotic or eukaryotic cells. Therefore, in another aspect, the invention is related to a host cell that has been transformed, transfected or infected with a vector provided by this invention. Said transformed, transfected or infected cell comprises, therefore, a gene construct of the invention, or said expression cassette or vector provided by this invention. Transformed, transfected or infected cells can be obtained by conventional methods known by persons skilled in the art [Sambrok et al., 1989, cited above]. In a particular embodiment, said host cell is an animal cell that has been transformed, transfected or infected with a suitable vector, said transformed, transfected or infected animal cell being capable of expressing the fusion protein provided by this invention, therefore said vectors can be used for expressing in animal cells the fusion protein provided by this invention.

The gene construct of he invention can be used to produce fusion proteins comprising (a) a polypeptide (A') comprising a region which recognizes and blocks a functionally active region of a molecule involved in the angiogenesis process; and (b) a polypeptide (B') comprising (i) an oligomerization domain, (ii) a functionally active region in the angiogenesis process, and (iii) a proteinase-sensitive region between said oligomerization domain and said functionally active region in the angiogenesis process.

Therefore, in another aspect, the invention is related to a method for producing said fusion protein provided by this invention, which comprises growing a cell or organism provided by this invention under conditions that allow the production of said fusion protein. The conditions for optimizing the culture of said cell or organism will depend on the cell or organism used. If so desired, the method for producing a product of interest provided by this invention further includes the isolation and purification of said fusion protein.

In another aspect, the invention is related to a fusion protein obtained by expression of the nucleic acid sequence contained in the gene construct of the invention. More specifically, the invention provides a fusion protein comprising:
(a) a polypeptide (A') comprising a region which recognizes and blocks a functionally active region of a molecule.involved in the angiogenesis process; and
(b) a polypeptide (B') comprising (i) an oligomerization domain, (ii) a functionally active region in the angiogenesis process, and (iii) a proteinase-sensitive region between said oligomerization domain and said functionally active region in the angiogenesis process.

Illustrative examples of molecules involved in the angiogenesis process include ECM proteins, angiogenic factors, cell membrane receptors, etc. Collagen, proteoglycans, fibronectin, laminin, tenascin, entactin and thrombospondin are among the ECM proteins. In a particular embodiment, said molecule involved in the angiogenesis process is a laminin, such as mammal laminin, for example rat, mouse or human laminin.

Virtually any polypeptide comprising a region which recognizes and blocks a functionally active region of a molecule involved in the angiogenesis process can be used in this invention, such as an antibody, for example a monoclonal antibody, that recognizes a molecule involved in the angiogenesis process, or a recombinant fragment thereof that recognizes said molecule involved in the angiogenic process, for example, a single chain Fv (scFv) fragment of an antibody that recognizes said molecule involved in the angiogenic process or a bi-specific antibody or diabody that recognizes said molecule involved in the angiogenic process or in its complete recombinant format (Fab + Fc); nevertheless, in a particular embodiment, said polypeptide (A') comprises a region which recognizes and blocks a functionally active region of a molecule involved in the angiogenesis process is a recombinant scFv derived from anti-laminin mAb L36 (Example 1) containing the variable heavy chain (VH) region of the monoclonal antibody L36 fused, through a linker, such as a peptide comprising the sequence G₄S, to the variable light-chain (VL) region of mAb L36 (Figure 2C), and whose sequence has been disclosed by Sanz L et al. [Cancer Immunology and Immunotherapy, 2001 Dec; 50(10)557-65], wherein the 3' end of the VL encoding sequence is bound to the 5' end of the sequence encoding said linker, and the 3' end of the nucleotide sequence encoding said linker is bound to the 5' end of the VL encoding sequence. As it is known, mAb L36 recognizes laminins from different animal species, for example mice, rats, humans, etc., since it interactions with a region that is very preserved between different animal species [Sanz L et al. EMBO J 2003, Vol. 22(7):1508-1517].

Said polypeptide (A') can recognize and block a functionally active region of a molecule involved in the angiogenesis process, and can direct an antiangiogenic peptide fused to said polypeptide to the molecules involved in the angiogenesis process, for example, ECM proteins, angiogenic factors, cell membrane receptors, etc., and block functionally active regions of said molecules

The polypeptide (B') comprises (i) an oligomerization domain, (ii) a functionally active region in the angiogenesis process, and (iii) a proteinase-sensitive region between said oligomerization domain and said functionally active region in the angiogenesis process. As previously mentioned, said oligomerization domain can be virtually any domain allowing oligomer formation, for example dimers, trimers, tetramers, etc., of peptides or proteins, capable of being expressed recombinantly and forming a proteinaceous oligomer of the protein comprising it. Nevertheless, in a particular embodiment, said oligomerization domain is a trimerization domain, such as the trimerization domain of the NC1 domain of mammal collagen XVIII or collagen XV.

The functionally active region in the angiogenesis process comprises any functionally active peptide or protein in the angiogenesis process, such as an angiogenesis process inhibitor or modulator peptide or protein. Virtually any functionally active peptide or protein in the angiogenesis process can be used in this invention; nevertheless, in a particular embodiment, said functionally active region in the angiogenesis process comprises a mammal endostatin (ES), such as the ES present in the NC1 domain of collagen XV or collagen XVIII.

The proteinase-sensitive region is located between said oligomerization domain (i) and said functionally active region in the angiogenesis process (ii), and comprises a peptide sequence susceptible to the action of a proteinase. Although virtually any peptide sequence susceptible to the action of any proteinase can be used in the present invention, said proteinase will advantageously be a proteinase expressed only in the tumor surroundings, so that the fusion protein of the invention will be "processed", generating monomers of functionally active peptides or proteins in the angiogenesis process (e.g., ES) and polypeptide trimeric molecules capable of recognizing and blocking a functionally active region of a molecule involved in the angiogenesis process (e.g., scFv). Therefore, in a particular embodiment, said proteinase-sensitive region comprises the hinge region present in the NC1 domain of mammal collagen XV or XVIII, between the trimerization domain and the ES region or domain of said NC1 domain, since said region is sensitive to the action of proteinases that are expressed only in the tumor surroundings.

As it is known, said NC1 domain of collagen XVIII (and collagen XV) comprises a trimerization domain and an ES domain bound by hinge peptides (Figure 2B). Therefore in a particular embodiment, the fusion protein of the invention comprises a polypeptide (B') comprising the NC1 domain of collagen XV or the NC1 domain of collagen XVIII containing the timerization and ES domains of said NC1 domains bound through the hinge peptides of said NC1 domains. The sequences of said NC1 domains of collagen XV and collagen XVIII are known; by way of illustration, the sequence of the NC1 domain of collagen XVIII has previously been disclosed by Sasaki et al. [Sasaki et al. Structure, function and tissue forms of the C-terminal globular domain of collagen XVIII containing the angiogenesis inhibitor endostatin. EMBO J. 1998 Aug 3;17(15):4249-56].

Therefore, by way of illustration, in a particular embodiment the invention provides a fusion protein comprising:
(i) a polypeptide selected from the group formed by whole mAb L36, mAb L36 in bifunctional format and a recombinant scFv containing the variable region of the heavy chain (VH) of the mAb L36 fused, through a flexible peptide, to the variable region of the light chain (VL) of mAb L36; and
(ii) a polypeptide (B') comprising the NC1 domain of mammal collagen XVIII.

However, any other domain similar to the NC1 domain of collagen XVIII comprising (i) an oligomerization domain, (ii) a functionally active region in the angiogenesis process, and (iii) a proteinase-sensitive region between said oligomerization domain and said functionally active region in the angiogenesis process, advantageously, a proteinase-sensitive region expressed in the tumor surroundings, can be used for putting this invention into practice, for the purpose of obtaining multifunctional and multivalent angiogenesis inhibitors.

The fusion protein provided by this invention may further contain, if so desired, a third polypeptide (C') comprising the amino acid sequence of a flexible binding peptide between said polypeptides (A') and (B') and/or (b) a peptide (D') to facilitate fusion protein isolation and purification.

As previously mentioned, said polypeptide (C') can comprise virtually any peptide sequence defining a flexible binding peptide. Illustrative examples of flexible binding peptides include sequences such as Gly-Ser-Pro-Gly or the sequence (Gly-Ser)₄. However, in a particular embodiment, said flexible binding peptide comprises the sequence Leu-Glu-Gly-Ala-Gly-Gly-Ser-Gly-Gly-Ser-Ser-Gly-Ser-Asp-Gly-Ala-Ser-Gly-Ser.

For the purpose of facilitating isolation and purification of the fusion protein of the invention, said fusion protein can likewise contain, if so desired, a peptide (D') capable of being used for the purpose of isolation or purification of the fusion protein, such as a tag peptide. Said peptide (D') can be located in any position of the fusion protein that does not alter the functionality of any of the polypeptides (A') and (B'), for example, said peptide (D') can be located after the polypeptide (B'). Virtually any peptide or peptide sequence allowing the isolation or purification of the fusion protein can be used, for example polyhistidine sequences, peptide sequences capable of being recognized by antibodies that may be useful for purifying the resultant fusion protein by immunoaffinity chromatography, such as tag peptides, etc., for example epitopes derived from the hemagglutinin of the fever virus or C-myc epitope, etc.

The fusion protein provided by this invention comprises a polypeptide (A') comprising a region which recognizes and blocks a functionally active region of a molecule involved in the angiogenesis process; and a polypeptide (B') comprising (i) an oligomerization domain, (ii) a functionally active region in the angiogenesis process, and (iii) a proteinase-sensitive region between said oligomerization domain and said functionally active region in the angiogenesis process. As a result of this fusion and due to the action of certain proteinases expressed only in the tumor surroundings, the fusion protein is "processed", generating monomers of functionally active peptides or proteins in the angiogenesis process (e.g., ES) and polypeptide trimeric molecules capable of recognizing and blocking a functionally active region of a molecule involved in the angiogenesis process (e.g., scFv). The invention thus provides multifunctional and multivalent angiogenesis inhibitor compounds since the angiogenesis process is attacked by several different paths in a joint manner.

Accordingly, due to its own features, the fusion protein of the invention can be used in treating and/or preventing the development of angiogenesis processes and, therefore, in treating and/or preventing pathologies occurring with angiogenesis processes.

Therefore, in another aspect, the invention is related to a pharmaceutical composition comprising either a fusion protein provided by this invention together with, at least, one pharmaceutically acceptable excipient, or a vector comprising a gene construct of the invention, or an expression cassette of the invention, and optionally at least one pharmaceutically acceptable excipient.

In a particular embodiment, the pharmaceutical composition of the invention comprises at least one fusion protein provided by this invention in a therapeutically effective quantity. In the sense used in this description, the expression "therapeutically effective quantity" refers to the quantity of fusion protein of the invention calculated to produce the desired effect and will generally be determined, among other reasons, by the own features of the fusion protein and the therapeutic effect to be obtained.

In another particular embodiment, the pharmaceutical composition provided by this invention is a composition intended for its use in gene therapy comprising a viral or nonviral vector provided by this invention comprising a gene construct of the invention or an expression cassette of the invention. By way of illustration, said vectors can be viral vectors, for example, based on retrovirus, adenovirus, etc., or nonviral such as DNA-liposome, DNA-polymer, DNA-polymer-liposome complexes, etc. [see "Nonviral Vectors for Gene Therapy", edited by Huang, Hung and Wagner, Academic Press (1999)]. Said vectors, which contain the gene construct of the invention or said expression cassette can be administered directly to the human or animal body by conventional methods. Said vectors may alternatively be used to transform, transfect or infect cells, for example mammal cells, including human cells, *ex vivo,* and, subsequently implant them in the human or animal body to obtain the desired therapeutic effect.

The pharmaceutical composition provided by this invention can be administered by any suitable administration method, for example orally or parenterally. The excipients that can be used in manufacturing the pharmaceutical composition provided by this invention will depend, among other factors, on the administration method of said pharmaceutical composition. A review of the different active ingredient administration methods, of the excipients to be used and of the processes for producing them can be found in the Tratado de Farmacia Galénica, C. Faulí i Trillo, Luzán 5, S.A. de Ediciones, 1993.

In another aspect, the invention is likewise related to the use of a fusion protein provided by this invention, or of a gene construct of the invention or an expression cassette provided by this invention or a recombinant vector provided by this invention, in the manufacture of a pharmaceutical composition to prevent, treat, impede or minimize angiogenesis development.

In another aspect, the invention is related to the use of a fusion protein provided by this invention, or of a gene construct of the invention or an expression cassette provided by this invention or a recombinant vector provided by this invention, in the manufacture of a pharmaceutical composition for treating and/or preventing pathologies occurring with angiogenesis. Illustrative, non-limiting examples of pathologies occurring with angiogenesis include cancer, hemangioma, rheumatic arthritis, psoriasis, bartonellosis, transplanted organ rejection, bleeding, ocular neovascularization (one of the most frequent cases of blindness), retinopathies (diabetic or early retinopathies, etc.), macular degeneration, neovascular glaucoma, retinal vein occlusion, retinal artery occlusion, pterygium, rubeosis, or corneal neovascularization. In a particular embodiment, the fusion protein of the invention, the gene construct of the invention, the expression cassette provided by this invention or the recombinant vector provided by this invention is particularly useful for treating cancer, including for treating solid tumors, and tumor proliferation and metastasis.

The following example can be used to illustrate the invention and must not be considered to be limiting of the scope thereof.

### EXAMPLE 1

### Design and expression and an angiogenesis inhibitor made up of an antibody fragment and collagen XVIII oligomers

Several gene constructs (Figure 3A) capable of expressing the following products have been generated:
- the single chain Fv fragment (scFv) of the anti-laminin monoclonal antibody L36 (L36 scFv),
- the fusion protein identified as L36 scFv-NC1^{ES-} made up of L36 scFv fused to the trimerization domain present in the NC1 domain of mouse collagen XVIII (residues 10 to 60) through a flexible connector peptide (linker),
- the fusion protein identified as L36 scFv-NC1^{ES+} made up of L36 scFv fused to the NC1 domain of mouse collagen XVIII (residues 10 to 325) through said linker,
- the NC1 domain of mouse collagen XVIII (residues 10 to 315) (NC1), and
- the endostatin (ES) from the NC1 domain of mouse collagen XVIII (residues 130 to 315), which is proteolytically released in trimeric form and subsequently converted into monomeric ES of approximately 20 kDa (Figures 2A and 2B).

For the purpose of facilitating immunodetection of the proteins, the gene constructs prepared further contained a sequence encoding a tag, specifically the sequence encoding his6myc, such that said tag was bound to the C-terminal end of the different proteins.

Said gene constructs were cloned into mammal expression vectors under the control of the human cytomegalovirus virus (hCMV) promoter containing the signal sequence of human oncostatin M [Sanz L et al Gene Therapy (2002) 15:1049]. Expression vectors for ES (positions 130-315) and mouse NC1 (1-315) were used as controls.

Said recombinant proteins were used in capillary structure formation assays in Matrigel as well as in cell migration assays for the purpose of evaluating their potential as an antiangiogenic agent.

### I. MATERIALS AND METHODS

### Cells and culture conditions

HEK-293 cells (human kidney epithelials; ATCC CRL-1573), HT-1080 cells (human fibrosarcoma; ATCC CCL-121) and B16-F10 cells (mouse melanoma; ATCC CRL-6475) were cultured in Dulbecco modified Eagle medium (DMEM) enriched with 10% of fetal bovine serum (FBS) (Life Technologies, Gaithersburg, MD, U.S.A.).

The primary human umbilical cord vein endothelial cells (HUVEC) were provided by Dr. B. Giménez (Instituto de Investigaciones Biomédicas, Madrid, Spain) and were cultured in Ham's F12K medium (Life Technologies) with 10% of FBS, 50 µg/ml of endothelial cell growth supplement (ECGS) coming form bovine pituitary and 100 µg/ml of heparin (Sigma Biosciences, St. Louis, MO, U.S.A.).

The human microvascular endothelial cell line HMEC-1 (Ades EW et al., 1992, Journal of Investigative Dermatology, 99:683-690) was supplied by Dr. E. W. Ades (Center for Disease Control, Atlanta, GA, U.S.A.) and was cultured in MCBD 131 medium (Life Technologies) enriched with 10% of FBS, 10 ng/ml of EGF (epidermal growth factor) and 1 mg/ml of hydrocortisone (Sigma Biosciences).

### Expression vector construction

The sequence of the NC1 domain, containing the sequence encoding the connector peptide, the trimerization domain, the hinge peptide and the ES domain (Figure 2B), was amplified by means of the polymerase chain reaction (PCR) from the al clone mc3b

(collagen XVIII) from mice [Oh et al. Genomics. 1994 Feb; 19(3):494-9] provided by Dr. B. Olsen (Harvard Medical School, Boston, MA, U.S.A.), with the primer pair 1 and 2 (see Table 1, containing the sequences of the different primers used for the vector construct and subsequent verification of the vector sequences).

**Table 1**

| | **Oligonucleotide sequences** |
|---|---|
| **Number** | **Sequence (5' →3')** |
| 1. | ATTCAGATCTTGGGCAGGTGAGGAT |
| 2. | TTCATGACCTCTTTCTCCAAAGCGGCCGCTAAACTAT |
| 3. | ATATAGCGCGGCCGCGAATTCAGATCTTGGGCAGGTGAGGAT |
| 4. | TAGAAGGCACAGTCGAGG |
| 5. | TCTGGCTCCAAGTCTGGC |
| 6. | AATTCAGGCGCCGGTGGATCTGGTGGCTCCTCTGGCTCAGACGGAGCGTC GGGTTCGCGA |
| 7. | GATCTCGCGAACCCGACGCTCCGTCTGAGCCAGAGGAGCCACCAGATCCA CCGGCGCCTG |
| 8. | CGATACA |
| 9. | GATCTGTAT |
| 10. | CCTAGATCTTGCTCATACTCATCAGGACTTTCAG |
| 11. | GTCCTAGGTGCGGCCGCGAAT |
| 12. | ATAGTTTAGCGGCCGCCTCATTGCCCGTGCCTCTCAG |

The PCR product was amplified again with the primer pair 2 and 3 (Table 1), and the PCR resultant product cleaved with NotI, was bound at the NotI site of the plasmid pCR3.1-L36 [Sanz L et al Gene Therapy (2002) 15:1049], to obtain the plasmid **pCR3.1-L36-NC1**. The sequence was checked using primers 4 and 5 (Table 1).

To generate the gene construct identified as L36 scFv-NC1^{ES+} (Figure 3A), the "connector peptide", connecting the collagen triple-helix with the NC1 trimerization domain, responsible for releasing the NC1 domain from parental collagen XVIII is replaced with a "flexible peptide connector" ("flexible linker") Leu-Glu-Gly-Ala-Gly-Gly-Ser-Gly-Gly-Ser-Ser-Gly-Ser-Asp-Gly-Ala-Ser-Gly-Ser [LEGAGGSGGSSGSDGASGS] (Figures 2B and 2C), for the purpose of preventing possible cleavage of the NC1 domain of the amino end of L36 scFv given that said "peptide connector" is sensitive to the action of different metalloproteinases (MMPs). To that end, a pair of oligonucleotides (primers 6 and 7) (Table 1) containing the sequence encoding said "flexible connector peptide" ("flexible linker") was bound at the EcoRI-BgIII site of.pCR3.1-L36-NC1, resulting in plasmid **pCR3.1-L36-linker-NC1.**

The plasmid **pCR3.1-NC1** was constructed by eliminating the ClaI-BglII fragment from the plasmid pCR3.1-L36-linker-NC1 (containing all the L36 scFv) and inserting a pair of oligonucleotides (primers 8 and 9) (Table 1) containing an NruI site.

To construct the plasmid **pCR3.1-ES,** the murine endostatin (ES) module (residues 130 to 315 of the NC1 domain) [Sasaki et al. Structure, function and tissue forms of the C-terminal globular domain of collagen XVIII containing the angiogenesis inhibitor endostatin. EMBO J. 1998 Aug 3;17(15):4249-56] was amplified from the plasmid pCR3.1-NC1 with the primer pair 2 and 10 (Table 1). The PCR fragment cleaved by BgIII/NotI was bound in the plasmid pCR3.1-NC1 digested by BgIII/NotI. The sequence was checked using primer 4 (Table 1).

The trimerization domain present in the NC1 domain [Sasaki et al. Structure, function and tissue forms of the C-terminal globular domain of collagen XVIII containing the angiogenesis inhibitor endostatin. EMBO J. 1998 Aug 3;17(15):4249-56] was amplified from the plasmid pCR3.1-NC1 with the primer pair 11 and 12 (Table 1). The PCR fragment digested with NotI was ligand to the plasmid pCR3.1-L36 digested with NotI, to obtain the plasmid pCR3.1-L36-Trimer. The sequence was checked using primer 4 (Table 1).

### Cell transfections

HEK-293, HT-1080 and B16-F10 cells were transfected with the suitable expression vectors (pCR3.1, pCR3.1-L36, pCR3.1-L36-Trimer, pCR3.1-ES, pCR3.1-NC1 or pCR3.1-L36-linker-NC1) using the system based on lipofectamine (Life Technologies). To generate stable cell lines, the transfected HEK-293, HT-1080 and B16-F10 cells were selected in DMEM with 0.5 mg/ml, 0.6 mg/ml or 3 mg/ml of G-418 (Life Technologies). The proliferation of parental tumor cells transduced by the various vectors was analyzed daily for 4 consecutive days in assays with 3-(4,5-dimethylthiazol-2-yl)-2,5-diphenyl tetrazoyl bromide (MTT, Promega). The supernatants of transitory and stable cell populations were analyzed to determine proteinaceous expression by means of SDS-PAGE [Sanz L et al. Gene Therapy (2002) 15:1049], ELISA [Sanz L. et al Gene Therapy (2002) 15:1049] and Western Blotting (immunoblotting) [Blanco et al. J. Immunol (2003) 171:1070] using anti-myc (Life Technologies) or anti-endostatin (Upstate Biotechnology, Lake Placid, NY, U.S.A.) mAbs (monoclonal antibodies).

### Expression and purification of recombinant proteins

Transfected stable HEK-293 cells were used to culture mediums conditioned without serum. A combination ("cocktail") of proteinase (aprotinin, bestatin, leupeptin, pepstatin A and E-64) inhibitors (Sigma Biosciences) was added to the medium to reduce proteolysis. The medium (approximately 1 L) was concentrated (x10) with a 10,000 MWCO Vivaflow 50 filter (Vivascience AG, Germany), dialyzed against PBS (phosphate buffered saline), pH 7.4 and loaded on a 1 mL HisTrap HP column (Amersham Biosciences, Uppsala, Sweden). The proteins containing ES were additionally purified on a 1 mL HiTrap Heparin HP column (Amersham Biosciences). The elution was carried out with a linear gradient of 0.1-2.0 NaCl. The purified proteins were dialyzed against PBS and stored at -20°C. The ES Fc-domain (Fc-ES) fusion protein [Cuo CJ et al. J Cell Biol. 2001 152:1233] was provided by Dr. K. Javaherian (Boston Children's Hospital, Boston, MA, U.S.A.). Said Fc-ES fusion protein is a dimeric fusion protein (between the ES domain and the Fc fragment of an immunoglobulin) and blocks capillary morphogenesis.

### Analytical centrifugation

Centrifugation experiments were carried out at 20°C in an Optima XL-A analytical ultracentrifuge (Beckman-Coulter Inc.) equipped with UV-visible optics using an An50Ti rotor with 3 mm double-sector centerpieces of Epon charcoal. The sedimentation balance at low speed was assayed in a short column (23 µl) at three successive speeds (5,000, 6,000 and 11,000 rpm), and images of the balance were taken (after 20 hours) at a wavelength of 280 nm. Attention was focused on the preservation of the proteinaceous mass in soluble form during the experiment. The sedimentation rate control experiments did not shown any aggregation in a 45-hour time interval. The reference signal was measured after high speed centrifugation (5 hours at 42,000 rpm). The average molecular weight by apparent weight of the whole cells was obtained by using the EQASSOC program [Minton, A. P. (1994) in Modern Analytical Ultracentrifugation (Schuster, T., and Laue, T., eds), pp. 81-93, Birkhauser Boston, Inc., Cambridge, MA]. The sedimentation rate experiment was carried out at 42,000 rpm and the absorbance images were taken at 280 nm. The sedimentation coefficients were calculated by means of the continuous distribution c(s) Lamm equation model [P. Schuck. Size-Distribution Analysis of Macromolecules by Sedimentation Velocity Ultracentrifugation and Lamm Equation Modeling. Biophysical Journal 78 (2000), pp. 1606-1619] as implemented in the SEDFIT program. These experimental sedimentation values were corrected for habitual conditions to obtain the corresponding *s*_{20,w} values using the SEDNTERP program [T.M. Laue, B.D. Shah, T.M. Ridgeway and S.L. Pelletier, Computer-aided interpretation of analytical sedimentation data for proteins. In: S.E. Harding, A.J. Rowe and J.C. Horton, Editors, Analytical Ultracentrifugation in Biochemistry and Polymer Science, Royal Society of Chemistry, Cambridge, UK (1992), pp. 90-125]. An additional hydrodynamic analysis (i.e. calculation of the frictional coefficient ratio) with the SEDFIT program to obtain the c(M) distribution [P. Schuck. Size-Distribution Analysis of Macromolecules by Sedimentation Velocity Ultracentrifugation and Lamm Equation Modeling. Biophysical Journal 78 (2000), pp. 1606-1619].

### Filtration chromatography on analytical gel

These experiments were carried out at room temperature with an ÄKTA FPLC system using a Superdex 200 10/300GL column: 0.1 mL samples of the different recombinant proteins (L36 scFv, L36 scFv-NC1^{ES-}, L36 scFv-NC1^{ES+} and NC1) in PBS were injected at a concentration of 0.5-1.0 mg/mL and were separated at a flow rate of 0.5 ml/min. The column was calibrated with high and low molecular weight markers (Amersham). The ratios of the elution volumes with respect to the exclusion volume and molecular weights of the markers were adjusted to an exponential curve which was used to calculate the molecular weight of the samples.

### Proteinases

The human MMP-1 and MMP-9 matrix metalloproteinases (MMP) were acquired from Oncogene Research Products (San Diego, CA, U.S.A.), while the human MMPs MMP-3, MMP-8 and MMP-14 (MT1-MMP) were acquired from Calbiochem (San Diego, CA, U.S.A.). Human cathepsin L and porcine pancreatic elastase were also acquired from Calbiochem.

### Proteolytic processing

The recombinant proteins L36 scFv-NC1^{ES-}, L36 scFv-NC1^{ES+} and NC1 were incubated at a concentration of 0.6 µM, at 37°C for 10 minutes with:
- 10 nM human cathepsin L in 50 mM sodium acetate, pH 5.5, dithiotreitol (DTT) 2 mM, 5 mM ethylenediaminetetraacetic acid (EDTA) (for treatment with human cathepsin L);
- 25 nM porcine elastase in 50 mM sodium acetate, pH 6.0 (for treatment with porcine elastase); and
- 25 nM metalloproteinases in 50 mM Tris-HC1, pH 7.5, 10 mM CaCl₂, 150 mM NaCl, 0.05% of Brij-35, 50 µM ZnSO₄ (for treatment with the metalloproteinases used).

Then aliquots were subjected to electrophoresis in 12% polyacrylamide gel under reducing conditions in the presence of sodium dodecylsulfate (SDS-PAGE). The gels were stained with silver nitrate (Sigma BioScience) or were subjected to Western Blot, using mouse anti-ES mAb.

### Tube formation assay in Matrigel

For capillary-like structure (CLS) formation assays, a Matrigel-based membrane matrix (Becton Dickinson, Bedford, MA, U.S.A.) was prepared on a pre-frozen 72-well Terasaki plate (Nunc, Roskilde, Denmark) with 2 µl/well and was then left to solidify at 37°C for 30 minutes. 3 x 10³ ECs (HMEC-1 or HUVEC) were seeded in 10 µl of suitable medium enriched with 0.5% FBS on the gelled matrix. The total volume of medium in the well was adjusted to 25 µl while at the same time the cells were prepared and were incubated for 16 hours at 37°C in an atmosphere moistened with 5% CO₂ The inhibitory effect of tube formation of the assayed recombinant proteins (L36 scFv, L36 scFv-NC1^{ES-}, L36 scFv-NC1^{ES+}, NC1 and ES) was determined by means of the addition of 15 µl of purified recombinant protein, diluted in the suitable medium according to the cell line used (HMEC-1 or HUVEC) at different concentrations (Figure 5D), to the Matrigel-coated plates at the time of plating the cells. The experiments were performed in triplicate.

### Digital image analysis

The digital images of each well were obtained from an Axiovert 100 microscope (Carl Zeiss, Germany), using a SPOT camera (Diagnostics Instruments, Inc.) and were stored as BMP images with 328x256 pixels 8 bit grey scale. The images were processed using the new AD software based on Matlab 5.3 [Sanz, L. et al. Microvasc Res. 2002 May; 63(3):335].

### Cell migration assay

For EC migration studies, polyethylene terephthalate filter inserts with a pore size of 3.0 µm and diameter of 6.5 mm (Becton Dickinson) were coated with Matrigel at a concentration of 1.25 µg/ml in serum-free medium at 4°C overnight. The HUVEC cells (1.5 x 10⁵) were preincubated in serum-free medium for 30 minutes at 37°C in 5% CO₂ atmosphere moistened in the presence of 20 µg/ml of purified protein (L36 scFv, L36 scFv-NC1^{ES-}, ES, NC1 and L36 scFv-NC1^{ES+}), and then they were transferred to the top compartment. 600 µl of medium containing serum were added to the bottom compartment. After 16 hours, the cells were fixed with 1% glutaraldehyde in PBS, were stained with 0.1 % violet crystal and examined under a microscope.

### II. RESULTS

### Combined effect of endostatin and a scFv of an anti-laminin antibody (L36) in the endothelial tube formation assay

It has been demonstrated that oligomeric ES modulates the extracellular matrix (ECM) dependent morphogenesis of epithelial cells (EC) [Cuo CJ et al. J Cell Biol. 2001 152:1233]. Treatment with dimeric ES, in Fc-ES form, at the time of plating the cells on Matrigel, significantly inhibited the assembly into tubular structures, keeping the EC dispersed and showing a morphology that was similar to that of the cells on plastic. These morphological changes were similar to those observed when the EC cultures were treated with L36 scFv (Figure 1).

### Design and expression of fusion proteins L36 scFv-ES

ES derives from the NC1 domain of collagen XVIII, which is proteolytically released in trimeric form and subsequently converted into monomeric ES of approximately 20 kDa (Figures 2A and 2B). Different chimeric genes expressing different proteins have been constructed.

In a particular embodiment, the chimeric gene called L36 scFv-NC1^{ES+} was constructed, made up of scFv of the anti-laminin mAb L36 fused to the NC1 domain of mouse collagen XVIII (residues 10 to 315) (Figures 2C and 3A). At the NC1 N-terminal end of collagen XVIII, there is a trimerization domain of about 60 amino acid residues approximately connected with the ES module, of about 180 amino acid residues, by means of a flexible hinge region of about 70 amino acid residues approximately, containing several sites that are sensitive to the proteinases which release the monomeric ES after the proteolytic cleavage (Figure 2B). The "connector peptide" connecting the collagen triple-helix with the NC1 trimerization domain responsible for releasing the NC1 domain from parental collagen XVIII is sensitive to the action of different MMPs. Therefore, to prevent a possible cleavage of the NC1 domain of the amino end of L36, said "connector peptide" was replaced by a "flexible linker" of 17 amino acids (Figures 2B and 2C).

Likewise, in another particular embodiment, the chimeric gene called L36 scFv-NC1^{ES-}, shorter than the previous one, containing the encoding sequences for L36 scFv, said flexible linker and only the trimerization domain of NC1, was constructed (Figure 3A).

The his6myc tag was attached at the C-terminal end of the fusion proteins to facilitate immunodetection.

The gene constructs were cloned into the suitable mammal expression vectors under the control of the hCMV promoter containing the main sequence of human M oncostatin. Expression vectors for ES (positions 130-315) and mouse NC1 (1-315) were used as a control (Figure 3A).

Stable cell lines were generated in HEK 293 cells and the fusion proteins of the conditioned medium were purified. The Western Blot analysis showed that the migration pattern of the purified proteins was consistent with the predicted molecular weight (Figure 3B) [lane 1: L36 scFv, lane 2: L36 scFv-NC1^{ES-}, lane 3: L36 scFv-NC1^{ES+}, lane 4: NC1, and lane 5: ES].

Under reducing conditions, the anti-myc mAb 9E10 developed single bands with an apparent molecular weight (MW) of 26, 36, 67.5 and 22 kDa (Figure 3B), which corresponded to the calculated MW of 28.8, 37.6, 65.6 and 23.8 kDa for monomeric L36 scFv, trimeric L36 scFv, L36 scFv-NC1 and monomeric ES, respectively. In the Western Blot analyses with proteins containing the NC1 domain, mAb 9E10 recognized a single band of approximately 24 kDa corresponding to monomeric ES and a band of a doublet of 39-44 kDa corresponding to the unprocessed protein (Figure 3B). Both bands within the doublet were positive in the Western Blot with and anti-ES mAb (Upstate Biotechnology, Lake Placid, NY 12946, USA). The doublet could be due to a post-translation modification or to internal protein folding effects.

The anti-ES mAb also developed the 24 kDa band and an additional band with an apparent MW of approximately 23 kDa which could correspond to unmarked ES-type monomers (Figure 3B). By means of Western Blot analysis with L36 scFv -NC1^{ES+} proteins, the anti-ES mAb recognized the unprocessed fusion of 67.5 kDa and a 24 kDa band not detected by means of the mAb 9E10, corresponding to the processed ES domain (Figure 3B). Purified ES appeared as a 22-23 kDa doublet when it was developed with the anti-ES mAb (Figure 3B).

### Characterization of fusion protein L36 scFv-NC1^{ES-}

The oligomerization status of the different fusion proteins was assessed by means of analytical chromatography with gel filtration and by means of analytical centrifugation experiments with identical samples. The fusion protein L36 scFv-NC1 ^{ES-} purified by means of IMAC (immobilized metal affinity chromatography) eluted from the gel filtration column essentially as a single peak (90% of the total area) at a volume that corresponded to a molecular weight of 109 kDa, which indicated that the protein was a trimer. The sedimentation rate also showed a main single peak with a mass of 112 kDa and the sedimentation equilibrium experience resulted in a mass distribution that could be adjusted to trimeric species and not to a monomer or a dimer (Figure 4A). As a whole, all these results showed the trimeric nature of the fusion protein L36 scFv-NC1^{ES-}, a characteristic conferred by the NC1 trimerization domain, since L36 scFv is monomeric under the same conditions as shown in the gel filtration and sedimentation rate experiments (data not shown). Trimeric L36 scFv (L36 scFv-NC1^{ES-}) showed greater binding affinity for laminin immobilized on plastic and was much more effective than the monomer in blocking EC differentiation on Matrigel substrates (Figures 4B and 4C).

For experimental purposes, it is essential that fusion protein L36 scFv-NC1^{ES-} remains stable and functional in the presence of proteinases. Therefore, its functionality after incubation with a wide variety of proteinases at known molar concentrations of active enzyme was determined. The proteinases included representatives of several classes of proteinases, i.e. cathepsin L of the cysteine proteinase family; MMP-1, MMP-3; MMP-8; MMP-9, MMP-14 of the metalloproteinase family, and pancreatic elastase of the serine proteinase family. As shown in Figure 4D, trimeric L36 scFv (L36 scFv-NC1^{ES-}) was not cleaved in the presence of the majority of the tested proteinases. MMP-14 partially processed the trimer (L36 scFv-NC1^{ES-}) but even after 4 hours of reaction, functionally active antibody was detected by means of ELISA. Only cathepsin L completely degraded the fusion protein L3 6 scFv-NC ^{ES-}.

### Characterization of fusion protein L36 scFv-NC1^{ES+}

Exclusion chromatography showed that purified fusion protein L36 scFv-NC1^{ES+} eluted as a main peak with an approximate molecular weight of 210 kDa, consistent with a trimer, and another peak with an apparent molecular weight of 117 kDa, which could correspond to a fragment lacking part of the ES (Figure 5A). The sedimentation rate also identified two species with weights of 181 kDa and 83 kDa.

The recombinant NC1 protein eluted as a main peak with an approximate molecular weight of 123 kDa, which corresponded with a trimeric species, and other smaller peaks, one of them with a weight of 19 kDa which corresponded with ES monomers (Figure 5B). A main species with a molecular weight of 116 kDa was identified by means of sedimentation rate experiments. This date indicated that the recombinant NC1 protein was primarily trimeric but heterogeneous, possibly due to degradation, which was consistent with the results obtained by other researchers who have observed a similar heterogeneity in recombinant NC1 proteins [Sasaki T. et al. EMBO J. 1998 17:4249-56].

As was expected, fusion protein L36 scFv-NC1^{ES+} showed binding affinity for laminin immobilized on plastic somewhat greater than that of the recombinant NC1 protein (Figure 5C). In the same manner, said fusion protein L36 scFv-NC1^{ES+} (bi-specific) was much more effective than the recombinant NC1 protein (monospecific) in blocking EC differentiation on Matrigel substrates (Figure 5D).

Figure 5E includes the results of the EC migration assay and shows that the migration of HUVEC cells in the presence of fusion protein L36 scFv-NC1^{ES+} is less than that reached in the presence of other assayed recombinant proteins (L36 scFv and NC1) and of the same order of magnitude as that reached with recombinant ES.

### Proteolytic processing of fusion protein L36 scFv-NC1^{ES+}. Monomeric ES and multimeric scFv generation.

In order to study ES generation by means of several proteinases, fusion protein L36 scFv-NC1^{ES+} was incubated and the reaction mixture was analyzed by means of Western Blot using an anti-ES mAb (Upstate Biotechnology, Lake Placid, NY 12946, USA). The fusion protein was treated with the five assayed MMPs (MMP-1, MMP-3, MMP-8, MMP-9 and MMP-14), significant differences being observed in the effectiveness of processing the protein L36 scFv-NC1^{ES+} between said MMPs, MMP-3 and MMP-14 being the most effective. The complete processing of fusion protein L36 scFv-NC1^{ES+} was observed in 4 hours. The main accumulation products were polypeptides with molecular weights comprised between 20 and 25 kDa (Figure 6A). MMP-3, MMP-8, MMP-9 and MMP-14 produced ES fragments that accumulated after 4 hours, which suggests that said MMPs cannot degrade them. As shown in Figure 6A, elastase and cathepsin L not only generated ES-type fragments from fusion protein L36 scFv-NC1^{ES+} but they also degraded them quickly, only small quantities of ES remaining after 4 hours of incubation. This proteolytic processing pattern was almost identical to that observed with the unmodified native murine NC1 protein (Figure 6B). The results indicated that the addition to the N-terminal end of an antibody fragment containing a site for recognizing an epitope, such as scFv, at the NC1 domain did not interfere with the proteolytic processing of the fusion protein by the proteinases nor with ES generation.

For the purpose of investigating the effectiveness of the proteinases for generating antibody fragments (L36 scFv), the reaction mixtures were subjected to SDS-PAGE under reducing conditions and were stained with silver nitrate. As shown in Figure 6C, the most effective proteinase in generating ES was MMP-14, which also generated L36 scFv. The generated L36 scFv and ES were stable and accumulated after 4 hours, which indicated that under these experimental conditions, the proteinase could not degrade them. The relationship between the intensity of trimeric L36 scFv and the intensity of the ES suggests a balanced processing of fusion protein L36 scFv-NC1^{ES+}. Similar results were observed with other MMPs (data not shown).

## Claims

1. An oligomeric protein comprising 2, 3, or 4 fusion proteins, wherein each fusion protein comprises:
a) a polypeptide (A') comprising a region which recognizes and blocks a functionally active region of a molecule involved in the angiogenesis process, said polypeptide being an antibody which recognizes and blocks a functionally active region of a molecule involved in the angiogenesis process or a recombinant fragment thereof which recognizes and blocks a functionally active region of a molecule involved in the angiogenesis process; and
b) a polypeptide (B') comprising (i) an oligomerization domain, (ii) an angiogenesis process inhibitor or modulator peptide or protein, and (iii) a proteinase-sensitive region between said oligomerization domain and said angiogenesis process inhibitor or modulator peptide or protein.

2. Oligomeric protein according to claim 1, wherein said molecule involved in the angiogenesis process is a protein of the extracellular matrix (ECM), an angiogenic factor or a cell membrane receptor.

3. Oligomeric protein according to claim 2, wherein said molecule involved in the angiogenesis process is collagen, a proteoglycan, fibronectin, laminin, tenascin, entactin, thrombospondin, vascular endothelial growth factor (VEGF), VEGF receptor 2 (VEGFR-2) or an integrin_{.}

4. Oligomeric protein according to claim 3, wherein said laminin is a mammal laminin, preferably a rat, mouse or human laminin.

5. Oligomeric protein according to anyone of claims 1 to 4, wherein said polypeptide (A') is a single chain Fv fragment (scFv) of an antibody which recognizes a molecule involved in the angiogenesis process or a diabody recognizing said molecule involved in the angiogenesis process.

6. Oligomeric protein according to claim 1, wherein said polypeptide (B') comprises the NC1 domain of mammal collagen XV or the NC1 domain of mammal collagen XVIII.

7. Oligomeric protein according to claim 1, comprising, between said polypeptides (A') and (B'), a third polypeptide (C') comprising the amino acid sequence of a flexible binding peptide.

8. Oligomeric protein according to claim 1, said oligomeric protein being a trimer consisting of 3 fusion proteins, wherein each fusion protein comprises:
a) a polypeptide (A'), said polypeptide (A') being a single chain Fv fragment (scFv) of an antibody which recognizes a laminin, and
b) a polypeptide (B'), said polypeptide (B') comprising the NC1 domain of mammal collagen XVIII.

9. A pharmaceutical composition comprising a oligomeric protein according to any of claims 1 to 8 together with at least one pharmaceutically acceptable excipient.

10. Use of an oligomeric protein according to any of claims 1 to 8, in the manufacture of a pharmaceutical composition to prevent, treat, impede or minimize angiogenesis development.

11. Use of an oligomeric protein according to any of claims 1 to 8, in the manufacture of a pharmaceutical composition for treating or preventing pathologies occurring with angiogenesis.

12. Use according to claim 11, wherein said pathology occurring with angiogenesis comprises cancer, hemangioma, rheumatic arthritis, psoriasis, bartonellosis, transplanted organ rejection, bleeding, ocular neovascularization, retinopathies, macular degeneration, neovascular glaucoma, retinal vein occlusion, retinal artery occlusion, pterygium, rubeosis, or corneal neovascularization.

13. An expression cassette comprising, operatively bound to a control sequence of the expression, a gene construct comprising, operatively bound, at least:
a) a first nucleic acid sequence (A), comprising the nucleotide sequence encoding a polypeptide which recognizes and blocks a functionally active region of a molecule involved in the angiogenesis process, said polypeptide being an antibody which recognizes and blocks a functionally active region of a molecule involved in the angiogenesis process or a recombinant fragment thereof which recognizes and blocks a functionally active region of a molecule involved in the angiogenesis process; and
b) a second nucleic acid sequence (B), comprising the nucleotide sequence encoding a polypeptide comprising (i) an oligomerization domain, (ii) an angiogenesis process inhibitor or modulator peptide or protein, and (iii) a proteinase-sensitive region between said oligomerization domain and said angiogenesis process inhibitor or modulator peptide or protein,
wherein the 3' end of said first nucleic acid sequence (A) is bound to the 5' end of said second nucleic acid sequence (B).

14. Expression cassette according to claim 13, wherein said molecule involved in the angiogenesis process is a protein of the extracellular matrix (ECM), an angiogenic factor or a cell membrane receptor.

15. Expression cassette according to claim 14, wherein said molecule involved in the angiogenesis process is collagen, a proteoglycan, fibronectin, laminin, tenascin, entactin, thrombospondin, vascular endothelial growth factor (VEGF), VEGF receptor 2 (VEGFR-2) or an integrin.

16. Expression cassette according to claim 15, wherein said laminin is a mammal laminin, preferably, a rat, mouse or human laminin.

17. Expression cassette according to claim 13, wherein said first nucleic acid sequence (A) comprises the nucleotide sequence encoding a single chain Fv fragment (scFv) of an antibody which recognizes said molecule involved in the angiogenesis process or a diabody which recognizes said molecule involved in the angiogenesis process.

18. Expression cassette according to claim 13, wherein said second nucleic acid sequence (B) comprises the nucleotide sequence encoding the NC1 domain of mammal collagen XV or the nucleotide sequence encoding the NC1 domain of mammal collagen XVIII, containing the nucleotide sequence corresponding to the trimerization domains and endostatin (ES) of said NC1 domains, and the nucleotide sequence encoding the hinge peptides of said NC1 domains.

19. Expression cassette according to claim 13, comprising, in addition to said nucleic acid sequences (A) and (B), a third nucleic acid sequence (C) containing the nucleotide sequence encoding a flexible binding protein, wherein the 5' end of said third nucleic acid sequence (C) is bound to the 3' end of said first nucleic acid sequence (A).

20. Expression cassette according to claim 13, wherein said first nucleic acid sequence (A) comprises the nucleotide sequence encoding a single chain Fv fragment (scFv) of an antibody which recognizes a laminin and said second nucleic acid sequence (B) comprises the nucleotide sequence encoding the NC1 domain of mammal collagen XVIII, containing the nucleotide sequence corresponding to the trimerization domain and endostatin (ES) of said NC1 domain.

21. A recombinant vector comprising an expression cassette according to any of claims 13 to 20.

22. A host cell comprising an expression cassette according to any of claims 13 to 20, or a recombinant vector according to claim 21.

23. An oligomeric protein obtained by expression of the nucleic acid sequence contained in an expression cassette according to any of claims 13 to 20.

24. A process for obtaining an oligomeric protein according to any of claims 1 to 8, comprising growing a cell according to claim 22 under conditions that allow the production of said oligomeric protein and, if so desired, isolating and purifying said Oligomeric protein.

25. A pharmaceutical composition comprising a vector comprising an expression cassette according to any of claims 13 to 20, together with at least one pharmaceutically acceptable excipient.

26. Use of an expression cassette according to any of claims 13 to 20, or of a vector according to claim 21, in the manufacture of a pharmaceutical composition to prevent, treat, impede or minimize angiogenesis development.

27. Use of an expression cassette according to any of claims 13 to 20, or of a vector according to claim 21, in the manufacture of a pharmaceutical composition for treating or preventing pathologies occurring with angiogenesis.

28. Use according to claim 27, wherein said pathology occurring with angiogenesis comprises cancer, hemangioma, rheumatic arthritis, psoriasis, bartonellosis, transplanted organ rejection, bleeding, ocular neovascularization, retinopathies, macular degeneration, neovascular glaucoma, retinal vein occlusion, retinal artery occlusion, pterygium, rubeosis, or corneal neovascularization.

## Patentansprüche

1. Oligomeres Protein, umfassend 2, 3 oder 4 Fusionsproteine, worin jedes Fusionsprotein umfasst:
(a) ein Polypeptid (A'), umfassend eine Region, die eine funktional aktive Region eines Moleküls, das an der Angiogenese beteiligt ist, erkennt und blockiert, wobei besagtes Polypeptid ein Antikörper ist, der eine funktional aktive Region eines Moleküls, das an der Angiogenese beteiligt ist, erkennt und blockiert, oder ein rekombinantes Fragment davon, das eine funktional aktive Region eines Moleküls, das an der Angiogenese beteiligt ist, erkennt und blockiert; und
(b) ein Polypeptid (B'), umfassend (i) eine Oligomerisationsdomäne, (ii) einen Angiogeneseinhibitor oder -modulator-Peptid oder -Protein und (iii) eine Proteinase-empfindliche Region zwischen der Oligomerisationsdomäne und dem Angiogeneseinhibitor oder -modulator-Peptid oder -Protein.

2. Oligomeres Protein gemäss Anspruch 1, worin das Molekül, das an der Angiogenese beteiligt ist, ein Protein der extrazellulären Matrix (ECM), ein angiogener Faktor oder ein Zellmembran-Rezeptor ist.

3. Oligomeres Protein gemäss Anspruch 2, worin das Molekül, das an der Angiogenese beteiligt ist, Kollagen, ein Proteoglycan, Fibronectin, Laminin, Tenascin, Entactin, Thrombospondin, ein vaskulärer endothelialer Wachstumsfaktor (VEGF), ein VEGF-Rezeptor 2 (VEGFR-2) oder ein Integrin ist.

4. Oligomeres Protein gemäss Anspruch 3, worin das Laminin ein Säuger-Laminin, vorzugsweise ein Ratten-, Mäuse- oder menschliches Laminin, ist.

5. Oligomeres Protein gemäss einem der Ansprüche 1 bis 4, worin das Polypeptid (A') ein einzelkettiges Fv-Fragment (scFv) eines Antikörpers ist, das ein Molekül erkennt, das an der Angiogenese beteiligt ist, oder einen Diabody, der das Molekül, das an der Angiogenese beteiligt ist, erkennt.

6. Oligomeres Protein gemäss Anspruch 1, worin das Polypeptid (B') die NC1-Domäne von Säuger-Kollagen XV oder die NC1-Domäne von Säuger-Kollagen XVIII umfasst.

7. Oligomeres Protein gemäss Anspruch 1, umfassend, zwischen den Polypeptiden (A') und (B'), ein drittes Polypeptid (C'), umfassend die Aminosäuresequenz eines flexiblen Bindungspeptids.

8. Oligomeres Protein gemäss Anspruch 1, das ein Trimer, bestehend aus 3 Fusionsproteinen, ist, worin jedes Fusionsprotein umfasst:
(a) ein Polypeptid (A'), das ein einzelkettiges Fv-Fragment (scFv) eines Antikörpers ist, das ein Laminin erkennt, und
(b) ein Polypeptid (B'), umfassend die NC1-Domäne von Säuger-Kollagen XVIII.

9. Pharmazeutische Zusammensetzung, umfassend ein oligomeres Protein gemäss einem der Ansprüche 1 bis 8 zusammen mit zumindest einem pharmazeutisch akzeptablen Hilfsstoff.

10. Verwendung eines oligomeren Proteins gemäss einem der Ansprüche 1 bis 8 in der Herstellung einer pharmazeutischen Zusammensetzung zum Verhindern, Behandeln, Hemmen oder Minimieren der Entwicklung von Angiogenese.

11. Verwendung eines oligomeren Proteins gemäss einem der Ansprüche 1 bis 8 in der Herstellung einer pharmazeutischen Zusammensetzung zur Behandlung oder Verhinderung von Pathologien, die mit Angiogenese auftreten.

12. Verwendung gemäss Anspruch 11, worin die Pathologie, die mit Angiogenese auftritt, Krebs, Hemangiom, rheumatoide Arthritis, Psoriasis, Bartonellose, Abstossung transplantierter Organe, Blutung, okulare Neovaskularisation, Retinopathien, Makuladegeneration, neovaskuläres Glaukom, retinalen Venenverschluss, retinalen Arterienverschluss, Pterygium, Rubeose oder Cornea-Neovaskularisation umfasst.

13. Expressionskassette, umfassend ein Genkonstrukt, das operativ mit einer Expressionskontrollsequenz verbunden ist, welches operativ verbunden zumindest umfasst:
(a) eine erste Nukleinsäuresequenz (A), umfassend die Nukleotidsequenz, codierend für ein Polypeptid, das eine funktional aktive Region eines Moleküls, das an der Angiogenese beteiligt ist, erkennt und blockiert, welches ein Antikörper ist, der eine funktional aktive Region eines Moleküls, das an der Angiogenese beteiligt ist, erkennt und blockiert, oder ein rekombinantes Fragment davon, das eine funktional aktive Region eines Moleküls, das an der Angiogenese beteiligt ist, erkennt und blockiert; und
(b) eine zweite Nukleinsäuresequenz (B), umfassend die Nukleinsäuresequenz, codierend für ein Polypeptid, umfassend (i) eine Oligomerisationsdomäne, (ii) einen Angiogeneseinhibitor oder -modulator-Peptid oder -Protein und (iii) eine Proteinase-empfindliche Region zwischen der Oligomerisationsdomäne und dem Angiogeneseinhibitor oder -modulator-Peptid oder -Protein,
worin das 3'-Ende der ersten Nukleinsäuresequenz (A) mit dem 5'-Ende der zweiten Nukleinsäuresequenz (B) verbunden ist.

14. Expressionskassette gemäss Anspruch 13, worin das Molekül, das an der Angiogenese beteiligt ist, ein Protein der extrazellulären Matrix (ECM), ein angiogener Faktor oder ein Zellmembran-Rezeptor ist.

15. Expressionskassette gemäss Anspruch 14, worin das Molekül, das an der Angiogenese beteiligt ist, Kollagen, ein Proteoglycan, Fibronectin, Laminin, Tenascin, Entactin, Thrombospondin, ein vaskulärer endothelialer Wachstumsfaktor (VEGF), ein VEGF-Rezeptor 2 (VEGFR-2) oder ein Integrin ist.

16. Expressionskassette gemäss Anspruch 15, worin das Laminin ein Säuger-Laminin, vorzugsweise ein Ratten-, Mäuse- oder menschliches Laminin, ist.

17. Expressionskassette gemäss Anspruch 13, worin die erste Nukleinsäuresequenz (A) die Nukleotidsequenz, codierend für ein einzelkettiges Fv-Fragment (scFv) eines Antikörpers, der das Molekül, das an der Angiogenese beteiligt ist, erkennt, oder einen Diabody, der das Molekül, das an der Angiogenese beteiligt ist, erkennt, umfasst.

18. Expressionskassette gemäss Anspruch 13, worin die zweite Nukleinsäuresequenz (B) die Nukleotidsequenz, codierend für die NC1-Domäne von Säuger-Kollagen XV, oder die Nukleotidsequenz, codierend für die NC1-Domäne von Säuger-Kollagen XVIII, enthaltend die Nukleotidsequenz, codierend für die Trimerisationsdomänen und Endostatin (ES) dieser NC1-Domänen, und die Nukleotidsequenz, codierend für die Gelenkpeptide dieser NC1-Domänen, umfasst.

19. Expressionskassette gemäss Anspruch 13, umfassend zusätzlich zu den Nukleinsäuresequenzen (A) und (B) eine dritte Nukleinsäuresequenz (C), enthaltend die Nukleotidsequenz, codierend für ein flexibles Bindungsprotein, worin das 5'-Ende dieser dritten Nukleinsäuresequenz (C) verbunden ist mit dem 3'-Ende der ersten Nukleinsäuresequenz (A).

20. Expressionskassette gemäss Anspruch 13, worin die erste Nukleinsäuresequenz (A) die Nukleotidsequenz, codierend für ein einzelkettiges Fv-Fragment (scFv) eines Antikörpers, der ein Laminin erkennt, umfasst und die zweite Nukleinsäuresequenz (B) die Nukleotidsequenz, codierend für die NC1-Domäne von Säuger-Kollagen XVIII, enthaltend die Nukleotidsequenz, die zu der Trimerisationsdomäne und Endostatin (ES) dieser NC1-Domäne korrespondiert, umfasst.

21. Rekombinanter Vektor, umfassend eine Expressionskassette gemäss einem der Ansprüche 13 bis 20.

22. Wirtszelle, umfassend eine Expressionskassette gemäss einem der Ansprüche 13 bis 20 oder einen rekombinanten Vektor gemäss Anspruch 21.

23. Oligomeres Protein, erhalten durch Expression der Nukleinsäuresequenz, enthalten in einer Expressionskassette gemäss einem der Ansprüche 13 bis 20.

24. Verfahren zur Gewinnung eines oligomeren Proteins gemäss einem der Ansprüche 1 bis 8, umfassend das Kultivieren einer Zelle gemäss Anspruch 22 unter Bedingungen, die die Produktion des oligomeren Proteins ermöglichen, und, falls erwünscht, Isolieren und Aufreinigen des oligomeren Proteins.

25. Pharmazeutische Zusammensetzung, umfassend einen Vektor, enthaltend eine Expressionskassette gemäss einem der Ansprüche 13 bis 20, zusammen mit zumindest einem pharmazeutisch akzeptablen Hilfsstoff.

26. Verwendung einer Expressionskassette gemäss einem der Ansprüche 13 bis 20 oder eines Vektors gemäss Anspruch 21 in der Herstellung einer pharmazeutischen Zusammensetzung zur Verhinderung, Behandlung, Hemmung oder Minimierung der Angiogeneseentwicklung.

27. Verwendung einer Expressionskassette gemäss einem der Ansprüche 13 bis 20 oder eines Vektors gemäss Anspruch 21 in der Herstellung einer pharmazeutischen Zusammensetzung zur Behandlung oder Verhinderung von Pathologien, die mit der Angiogenese auftreten.

28. Verwendung gemäss Anspruch 27, worin die Pathologie, die mit Angiogenese auftritt, Krebs, Hemangiom, rheumatoide Arthritis, Psoriasis, Bartonellose, Abstossung transplantierter Organe, Blutung, okulare Neovaskularisation, Retinopathien, Makuladegeneration, neovaskuläres Glaukom, retinalen Venenverschluss, retinalen Arterienverschluss, Pterygium, Rubeose oder Cornea-Neovaskularisation umfasst.

## Revendications

1. Protéine oligomérique comprenant 2, 3 ou 4 protéines de fusion **caractérisées en ce que** chaque protéine de fusion comprend :
a) un polypeptide (A') comprenant une région qui reconnaît et bloque une région fonctionnellement active d'une molécule impliquée dans le processus d'angiogenèse, le dit polypeptide étant un anticorps qui reconnaît et bloque une région fonctionnellement active d'une molécule impliquée dans le processus d'angiogenèse ou un fragment recombinant de celui-ci qui reconnaît et bloque une région fonctionnellement active d'une molécule impliquée dans le processus d'angiogenèse, et
b) un polypeptide (B') comprenant (i) un domaine d'oligomérisation, (ii) un peptide ou une protéine inhibant ou modulant le processus d'angiogenèse, et (iii) une région sensible à la protéinase entre le dit domaine d'oligomérisation et le dit peptide ou protéine inhibant ou modulant le processus d'angiogenèse.

2. Protéine oligomérique selon la revendication 1, **caractérisée en ce que** la dite molécule impliquée dans le processus d'angiogenèse est une protéine de la matrice extracellulaire (ECM), un facteur angiogénique ou un récepteur de la membrane cellulaire.

3. Protéine oligomérique selon la revendication 2, **caractérisée en ce que** la dite molécule impliquée dans le processus d'angiogenèse est le collagène, un protéoglycane, la fibronectine, la laminine, la ténascine, l'entactine, la thrombospondine, le facteur de croissance endothélial vasculaire (VEGF), le récepteur 2 du VEGF (VEGFR-2) ou une intégrine.

4. Protéine oligomérique selon la revendication 3, **caractérisée en ce que** la dite laminine est une laminine de mammifère, de préférence une laminine de rat, de souris ou humaine.

5. Protéine oligomérique selon l'une quelconque des revendications 1 à 4, **caractérisée en ce que** le dit polypeptide (A') est un fragment Fv simple chaîne (scFv) d'un anticorps qui reconnaît une molécule impliquée dans le processus d'angiogenèse ou un diacorps reconnaissant la dite molécule impliquée dans le processus d'angiogenèse.

6. Protéine oligomérique selon la revendication 1, **caractérisée en ce que** le dit polypeptide (B') comprend le domaine NC1 du collagène XV de mammifère ou le domaine NC1 du collagène XVIII de mammifère.

7. Protéine oligomérique selon la revendication 1, comprenant, entre les dits polypeptides (A') et (B'), un troisième polypeptide (C') comprenant la séquence d'acides aminés d'un peptide de liaison flexible.

8. Protéine oligomérique selon la revendication 1, la dite protéine oligomérique étant un trimère composé de 3 protéines de fusion, **caractérisée en ce que** chaque protéine de fusion comprend :
a) un polypeptide (A'), le dit polypeptide (A') étant un fragment Fv simple chaîne (scFv) ou un anticorps qui reconnaît une laminine, et
b) un polypeptide (B'), le dit polypeptide (B') comprenant le domaine NC1 du collagène XVIII de mammifère.

9. Composition pharmaceutique comprenant une protéine oligomérique selon l'une quelconque des revendications 1 à 8 avec au moins un excipient pharmaceutiquement acceptable.

10. Utilisation d'une protéine oligomérique selon l'une quelconque des revendications 1 à 8, dans la fabrication d'une composition pharmaceutique pour empêcher, traiter, gêner ou minimiser le développement de l'angiogenèse.

11. Utilisation d'une protéine oligomérique selon l'une quelconque des revendications 1 à 8, dans la fabrication d'une composition pharmaceutique pour traiter ou empêcher des pathologies survenant avec l'angiogenèse.

12. Utilisation selon la revendication 11, **caractérisée en ce que** la dite pathologie survenant avec l'angiogenèse comprend le cancer, l'hémangiome, la polyarthrite rhumatoïde, le psoriasis, la bartonellose, le rejet des organes transplantés, les saignements, la néovascularisation oculaire, les rétinopathies, la dégénérescence maculaire, le glaucome néovasculaire, l'occlusion des veines rétiniennes, l'occlusion des artères rétiniennes, le ptérygion, la rubéose ou la néovascularisation cornéenne.

13. Cassette d'expression comprenant, liée de manière fonctionnelle à une séquence de contrôle de l'expression, une construction génique comprenant, liées de manière fonctionnelle, au moins :
a) une première séquence d'acide nucléique (A), comprenant la séquence nucléotidique encodant un polypeptide qui reconnaît et bloque une région fonctionnellement active d'une molécule impliquée dans le processus d'angiogenèse, le dit polypeptide étant un anticorps qui reconnaît et bloque une région fonctionnellement active d'une molécule impliquée dans le processus d'angiogenèse ou un fragment recombinant de celui-ci qui reconnaît et bloque une région fonctionnellement active d'une molécule impliquée dans le processus d'angiogenèse, et
b) une deuxième séquence d'acide nucléique (B), comprenant la séquence nucléotidique encodant un polypeptide comprenant (i) un domaine d'oligomérisation, (ii) un peptide ou une protéine inhibant ou modulant le processus d'angiogenèse, et (iii) une région sensible à la protéinase entre le dit domaine d'oligomérisation et le dit peptide ou protéine inhibant ou modulant le processus d'angiogenèse,
**caractérisée en ce que** l'extrémité 3' de la dite première séquence d'acide nucléique (A) est liée à l'extrémité 5' de la dite deuxième séquence d'acide nucléique (B).

14. Cassette d'expression selon la revendication 13, **caractérisée en ce que** la dite molécule impliquée dans le processus d'angiogenèse est une protéine de la matrice extracellulaire (ECM), un facteur angiogénique ou un récepteur de la membrane cellulaire.

15. Cassette d'expression selon la revendication 14, **caractérisée en ce que** la dite molécule impliquée dans le processus d'angiogenèse est le collagène, un protéoglycane, la fibronectine, la laminine, la ténascine, l'entactine, la thrombospondine, le facteur de croissance endothélial vasculaire (VEGF), le récepteur 2 du VEGF (VEGFR-2) ou une intégrine.

16. Cassette d'expression selon la revendication 15, **caractérisée en ce que** la dite laminine est une laminine de mammifère, de préférence une laminine de rat, de souris ou humaine.

17. Cassette d'expression selon la revendication 13, **caractérisée en ce que** la dite première séquence d'acide nucléique (A) comprend la séquence nucléotidique encodant un fragment Fv simple chaîne (scFv) d'un anticorps qui reconnaît la dite molécule impliquée dans le processus d'angiogenèse ou un diacorps qui reconnaît la dite molécule impliquée dans le processus d'angiogenèse.

18. Cassette d'expression selon la revendication 13, **caractérisée en ce que** la dite deuxième séquence d'acide nucléique (B) comprend la séquence nucléotidique encodant le domaine NC1 du collagène XV de mammifère ou la séquence nucléotidique encodant le domaine NC1 du collagène XVIII de mammifère, contenant la séquence nucléotidique correspondant aux domaines de trimérisation et à l'endostatine (ES) des dits domaines NC1, et la séquence nucléotidique encodant les peptides d'articulation des dits domaines NC1.

19. Cassette d'expression selon la revendication 13, comprenant en plus des dites séquences d'acide nucléique (A) et (B), une troisième séquence d'acide nucléique (C) contenant la séquence nucléotidique encodant une protéine de liaison flexible, **caractérisée en ce que** l'extrémité 5' de la dite troisième séquence d'acide nucléique (C) est liée à l'extrémité 3' de la dite première séquence d'acide nucléique (A).

20. Cassette d'expression selon la revendication 13, **caractérisée en ce que** la dite première séquence d'acide nucléique (A) comprend la séquence nucléotidique encodant un fragment Fv simple chaîne (scFv) d'un anticorps qui reconnaît une laminine et la dite deuxième séquence d'acide nucléique (B) comprend la séquence nucléotidique encodant le domaine NC1 du collagène XVIII de mammifère, contenant la séquence nucléotidique correspondant au domaine de trimérisation et à l'endostatine (ES) du dit domaine NC1.

21. Vecteur recombinant comprenant une cassette d'expression selon l'une quelconque des revendications 13 à 20.

22. Cellule hôte comprenant une cassette d'expression selon l'une quelconque des revendications 13 à 20, ou un vecteur recombinant selon la revendication 21.

23. Protéine oligomérique obtenue par l'expression de la séquence d'acide nucléique contenue dans une cassette d'expression selon l'une quelconque des revendications 13 à 20.

24. Processus pour obtenir une protéine oligomérique selon l'une quelconque des revendications 1 à 8, comprenant la croissance d'une cellule selon la revendication 22 dans des conditions qui permettent la production de la dite protéine oligomérique et, si désiré, l'isolement et la purification de la dite protéine oligomérique.

25. Composition pharmaceutique comprenant un vecteur comprenant une cassette d'expression selon l'une quelconque des revendications 13 à 20, avec au moins un excipient pharmaceutiquement acceptable.

26. Utilisation d'une cassette d'expression selon l'une quelconque des revendications 13 à 20, ou d'un vecteur selon la revendication 21, dans la fabrication d'une composition pharmaceutique pour empêcher, traiter, gêner ou minimiser le développement de l'angiogenèse.

27. Utilisation d'une cassette d'expression selon l'une quelconque des revendication 13 à 20, ou d'un vecteur selon la revendication 21, dans la fabrication d'une composition pharmaceutique pour traiter ou empêcher les pathologies survenant avec l'angiogenèse.

28. Utilisation selon la revendication 27, **caractérisée en ce que** la dite pathologie survenant avec l'angiogenèse comprend le cancer, l'hémangiome, la polyarthrite rhumatoïde, le psoriasis, la bartonellose, le rejet des organes transplantés, les saignements, la néovascularisation oculaire, les rétinopathies, la dégénérescence maculaire, le glaucome néovasculaire, l'occlusion des veines rétiniennes, l'occlusion des artères rétiniennes, le ptérygion, la rubéose ou la néovascularisation cornéenne.
